# EUROPEAN PATENT APPLICATION

(11) **EP 3 556 362 A1**
(43) Date of publication of application: **23.10.2019**
(21) Application number: 18167592.7
(22) Date of filing: 16.04.2018
(51) Int. Cl.: A61K 31/404, A61P 25/00, A61P 29/00, A61P 37/00

(54) **STING INHIBITORS**

(71) Applicant: ECOLE POLYTECHNIQUE FEDERALE DE LAUSANNE (EPFL), 1015 Lausanne (CH)
(72) Inventor: ABLASSER, Andrea, 1091 Aran (CH); HAAG, Simone, 1026 Echandens-Denges (CH); REYMOND, Luc, 1806 St-Légier (CH)
(74) Representative: KATZAROV S.A.

(57) **Abstract**

The present invention relates to compounds, compositions for use and methods for the treatment of a STING associated disease, condition, or disorder.

SEQUENCE LISTING

<110>  ECOLE POLYTECHNIQUE FEDERALE DE LAUSANNE (EPFL)

<120>  STING inhibitors

<130>  51261/EP

<160>  4

<170>  PatentIn version 3.5

<210>  1
<211>  20
<212>  DNA
<213>  Artificial Sequence

<220>
<223>  synthetic sequence

<400>  1
gagtcaacgg atttggtcgt                                                   20


<210>  2
<211>  20
<212>  DNA
<213>  Artificial Sequence

<220>
<223>  synthetic sequence

<400>  2
gacaagcttc ccgttctcag                                                   20


<210>  3
<211>  20
<212>  DNA
<213>  Artificial Sequence

<220>
<223>  synthetic sequence

<400>  3
cagcatctgc tggttgaaga                                                   20


<210>  4
<211>  20
<212>  DNA
<213>  Artificial Sequence

<220>
<223>  synthetic sequence

<400>  4
cattacctga aggccaagga                                                   20

## Description

### FIELD OF THE INVENTION

The present invention relates to compounds, compositions for use and methods for the treatment of a STING associated disease, condition, or disorder.

### BACKGROUND OF THE INVENTION

STING (Stimulator of Interferon Genes) is an innate signalling molecule that is pivotal in mediating the recognition of cytosolic DNA^{1,2}. In this context, STING, a transmembrane protein localized to the endoplasmic reticulum (ER), acts as a second messenger receptor for 2', 3' cyclic GMP-AMP (hereafter cGAMP), which is produced by cGAS after dsDNA binding³⁻⁷. In addition, STING can also function as a primary pattern recognition receptor for bacterial cyclic dinucleotides (CDNs) and small molecule agonists ^{8,9,10}. The recognition of endogenous or prokaryotic CDNs proceeds through the carboxy-terminal domain of STING, which faces into the cytosol and creates a V-shaped binding pocket formed by a STING homodimer¹¹⁻¹⁴. Ligand-induced activation of STING triggers its re-localization to the Golgi, a process essential to promote the interaction of STING with TBK1¹⁵. This protein complex, in turn, signals through the transcription factors IRF-3 to induce type I interferons (IFNs) and other co-regulated antiviral factors¹⁶. In addition, STING was shown to trigger NF-• B and MAP kinase activation^{17,18}. Following the initiation of signal transduction, STING is rapidly degraded, a step considered important in terminating the inflammatory response¹⁹.

Excessive activation of STING is associated with a subset of monogenic autoinflammatory conditions, the so-called type I interferonopathies²⁰. Examples of these diseases include a clinical syndrome referred to as STING-associated vasculopathy with onset in infancy (SAVI), which is caused by gain-of-function mutations in TMEM173 (the gene name of STING)^{21,22}. Moreover, STING is implicated in the pathogenesis of Aicardi-Goutières Syndrome (AGS) and genetic forms of lupus²³⁻²⁵. As opposed to SAVI, it is the dysregulation of nucleic acid metabolism that underlies continuous innate immune activation in AGS. Apart from these genetic disorders, emerging evidence points to a more general pathogenic role for STING in a range of inflammation-associated disorders such as systemic lupus erythematosus, rheumatoid arthritis and cancer^{26,27}. Thus, small molecule-based pharmacological interventions into the STING signalling pathway hold significant potential for the treatment of a wide spectrum of diseases.

### BRIEF DESCRIPTION OF THE FIGURES

**Figure 1****|** Chemical structure of F2740-0151
**Figure 2****|** HEK293T cells were transfected with vectors encoding for hsSTING or mmSTING in combination with cGAS, TBK1, mmSTING V154M or RIG-I and a IFN-• luciferase reporter, respectively, and treated with HI. (concentration of the compounds: 0.31-2.5µM). Reporter activity was measured 16 h after compound treatment.
**Figure 3****|** Differentiated THP-1 cells were pretreated with DMSO or F2740-0151 (0.5 µM, 1 µM) as indicated for 90 mins. Subsequently, cells were stimulated with cGAMP or triphosphate RNA for 3 h. RT-qPCR was performed to assess the induction of IFNB1 and TNFa, respectively.
**Figure 4****|** Deconvoluted ESI mass spectrum showing intact mass of Flag-hsSTING(left) and the Flag-hsSTING-Hl complex (right).
**Figure 5****|** Deconvoluted ESI mass spectrum showing intact mass of Flag-hsSTING C91S (left) and the Flag-hsSTING C91S treated with H1 (right).
**Figure 6****|** Labelling of recombinant Flag-hsSTING in HEK293T cells with BSF051 in indicated concentrations.
**Figure 7****|** Labelling of indicated STING constructs in HEK293T cells with BSF051 (0.25 µM).
**Figure 8****|** A: Chemical structure of BSF0061 BSF0062 and BSF0063 B: HEK293T cells were transfected with vectors encoding for hsSTING in combination with cGAS, RIG-I and a IFN-• luciferase reporter, respectively, and treated with F2740-0151. (concentration of the compounds: 0.31-2.5µM). Reporter activity was measured 16 h after compound treatment.

### SUMMARY OF THE INVENTION

The present invention provides compounds presenting formula (I): 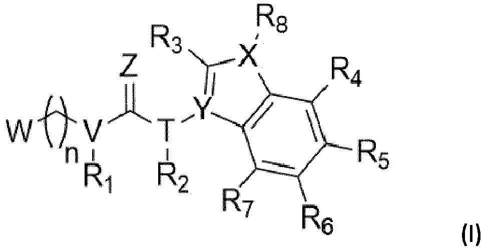 racemates, enantiomers, diastereomers and pharmaceutically acceptable salts thereof, wherein
R¹ and R² are each independently selected from H, C₁-C₁₀ alkyl, C₂-C₆ alkenyl, C₁-C₁₀ haloalkyl or a lone pair of electrons;
R³ is H;
R⁴, R⁵, R⁶ and R⁷ are each independently selected from H, C₁-C₁₀ alkyl, C₂-C₁₀ alkenyl, C₂-C₁₀ alkynyl, C₁-C₁₀ haloalkyl, C₁-C₁₀ alkoxy, C₁-C₁₀ carboxyalkyl, carboxyl, halogen, nitro, cyano, amino, amido, or ether groups;
R⁸ is selected from H, C₁-C₆ alkyl, C₂-C₆ alkenyl, C₂-C₁₀ alkynyl or C₁-C₆ haloalkyl;
X is selected from O, S, or NH;
Z is selected from O, S, or N;
Y is C, O, S or N;
T and V are independently selected from O, S, or N;
W is selected from saturated or unsaturated C₁-C₂₀ alkyl group (preferably C₆-C₂₀ alkyl group), C₆-C₃₁ aryl group, C₆-C₃₁ heteroaryl group, C₅-C₃₁ aryloxy group, or C₅-C₃₁ aralkyl group, wherein each group is optionally substituted with 1, 2, 3 substituents independently selected from -O-CF₃, -CF₃, -O-CHF₂, halogen, -OH, -NH₂, -CN, -OCH₃, C₁-C₆ alkyl, allyl, -C(O)OR¹, -C(O)NH₂, propargyl, -NO₂, -S(O)₂NH₂, and -S(O)CH₃;
and n is an integer between 0-2, with the proviso that 1-(4-ethylphenyl)-3-(1H-indol-3-yl)urea is excluded.

A further object of the present invention is to provide compound having formula I: 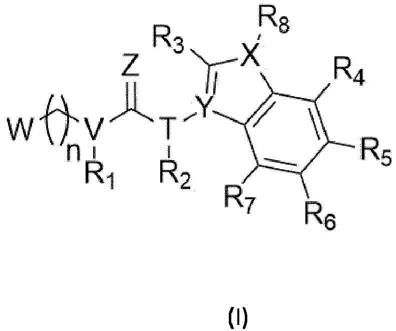 racemates, enantiomers, diastereomers and pharmaceutically acceptable salts thereof, wherein
R¹ and R² are each independently selected from H, C₁-C₁₀ alkyl, C₂-C₆ alkenyl, C₁-C₁₀ haloalkyl or a lone pair of electrons;
R³ is H;
R⁴, R⁵, R⁶ and R⁷ are each independently selected from H, C₁-C₁₀ alkyl, C₂-C₁₀ alkenyl, C₂-C₁₀ alkynyl, C₁-C₁₀ haloalkyl, C₁-C₁₀ alkoxy, C₁-C₁₀ carboxyalkyl, carboxyl, halogen, nitro, cyano, amino, amido, or ether groups;
R⁸ is selected from H, C₁-C₆ alkyl, C₂-C₆ alkenyl, C₂-C₁₀ alkynyl or C₁-C₆ haloalkyl;
X is selected from O, S, or NH;
Z is selected from O, S, or N;
Y is C, O, S or N;
T and V are independently selected from O, S, or N;
W is selected from saturated or unsaturated C₁-C₂₀ alkyl group, C₆-C₃₁ aryl group, C₆-C₃₁ heteroaryl group, C₅-C₃₁ aryloxy group, or C₅-C₃₁ aralkyl group, wherein each group is optionally substituted with 1, 2, 3 substituents independently selected from -O-CF₃, -CF₃, -O-CHF₂, halogen, -OH, -NH₂, -CN, -OCH₃, C₁-C₆ alkyl, allyl, -C(O)OR¹, -C(O)NH₂, propargyl, -NO₂, -S(O)₂NH₂, and -S(O)CH₃; and n is an integer between 0-2, for use in the treatment of a STING associated disease.

A further object of the present invention concerns a pharmaceutical composition comprising a compound of formula (I): 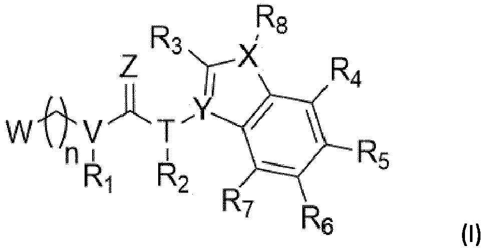 racemates, enantiomers, diastereomers and pharmaceutically acceptable salts thereof, wherein
R¹ and R² are each independently selected from H, C₁-C₁₀ alkyl, C₂-C₆ alkenyl, C₁-C₁₀ haloalkyl or a lone pair of electrons;
R³ is H;
R⁴, R⁵, R⁶ and R⁷ are each independently selected from H, C₁-C₁₀ alkyl, C₂-C₁₀ alkenyl, C₂-C₁₀ alkynyl, C₁-C₁₀ haloalkyl, C₁-C₁₀ alkoxy, C₁-C₁₀ carboxyalkyl, carboxyl, halogen, nitro, cyano, amino, amido, or ether groups;
R⁸ is selected from H, C₁-C₆ alkyl, C₂-C₆ alkenyl, C₂-C₁₀ alkynyl or C₁-C₆ haloalkyl;
X is selected from O, S, or NH;
Z is selected from O, S, or N;
Y is C, O, S or N;
T and V are independently selected from O, S, or N;
W is selected from saturated or unsaturated C₁-C₂₀ alkyl group, C₆-C₃₁ aryl group, C6-C₃₁ heteroaryl group, C₅-C₃₁ aryloxy group, or C₅-C₃₁ aralkyl group, wherein each group is optionally substituted with 1, 2, 3 substituents independently selected from -O-CF₃, -CF₃, -O-CHF₂, halogen, -OH, -NH₂, -CN, -OCH₃, C₁-C₆ alkyl, allyl, -C(O)OR¹, -C(O)NH₂, propargyl, -NO₂, -S(O)₂NH₂, and -S(O)CH₃; and n is an integer between 0-2, and a pharmaceutically acceptable excipient.

The present invention also concerns the use of a compound of the invention having formula (I): 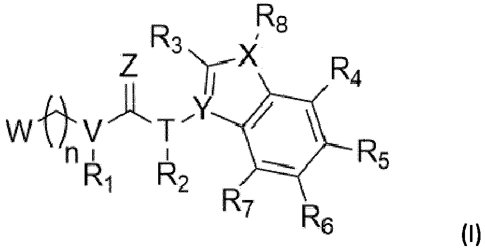 racemates, enantiomers, diastereomers and pharmaceutically acceptable salts thereof, wherein
R¹ and R² are each independently selected from H, C₁-C₁₀ alkyl, C₂-C₆ alkenyl, C₁-C₁₀ haloalkyl or a lone pair of electrons;
R³ is H;
R⁴, R⁵, R⁶ and R⁷ are each independently selected from H, C₁-C₁₀ alkyl, C₂-C₁₀ alkenyl, C₂-C₁₀ alkynyl, C₁-C₁₀ haloalkyl, C₁-C₁₀ alkoxy, C₁-C₁₀ carboxyalkyl, carboxyl, halogen, nitro, cyano, amino, amido, or ether groups;
R⁸ is selected from H, C₁-C₆ alkyl, C₂-C₆ alkenyl, C₂-C₁₀ alkynyl or C₁-C₆ haloalkyl;
X is selected from O, S, or NH;
Z is selected from O, S, or N;
Y is C, O, S or N;
T and V are independently selected from O, S, or N;
W is selected from saturated or unsaturated C₁-C₂₀ alkyl group, C₆-C₃₁ aryl group, C₆-C₃₁ heteroaryl group, C₅-C₃₁ aryloxy group, or C₅-C₃₁ aralkyl group, wherein each group is optionally substituted with 1, 2, 3 substituents independently selected from -O-CF₃, -CF₃, - O-CHF₂, halogen, -OH, -NH₂, -CN, -OCH₃, C₁-C₆alkyl, allyl, -C(O)OR¹, -C(O)NH₂, propargyl, -NO₂, -S(O)₂NH₂, and -S(O)CH₃; and n is an integer between 0-2, or pharmaceutical composition of the invention, in the manufacture of a medicament.

The present invention further concerns a kit comprising (1) a pharmaceutical composition of the invention, and (2) instructions for use.

A method of treatment and/or prevention of a STING associated disease comprising administering a pharmaceutical composition of the invention is also envisioned.

### DESCRIPTION OF THE INVENTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

In some aspects, the compounds described herein inhibit the signaling molecule STING by binding at least one amino acid of a region including the amino acid residues 85 to 95. The binding can be covalent or non-covalent. Preferably, the binding is a covalent binding. Most preferably, the compound covalently binds to the transmembrane-located cysteine, C91.
It is believed that, by acting through, preferably, covalent binding to a transmembrane-located cysteine, C91, the compounds of the invention exploit an unanticipated mechanism in order to attain potency and selectivity against STING. In the absence of inhibition, ligand binding triggers the translocation of STING to the Golgi, where palmitoylation occurs at luminal proximal cysteine residues (C91 and C88)^{15 31}. This post-translational modification, in turn, facilitates the multimerisation of STING creating a platform for the recruitment of TBK1 - and possibly other adaptor molecules - and thereby enabling the initiation of further downstream signalling ^{29 16}. Through covalent interaction with C91, the compounds of the invention and in particular C-178 and C-176 block palmitoylation of STING and retain the protein in a signalling incompetent state. Of major significance, the palmitate-modified cysteines are highly conserved residues. This provides confidence that the mechanistic insight of small molecule-mediated antagonism of STING delineated here will be useful for the development of next-generation inhibitors of STING that are active in humans. For now, C-178 and C-176 provide a powerful new tool to acutely perturb STING *in vitro* and offer the unique possibility to pharmacologically intervene into the function of STING *in vivo.* Along these considerations, previous studies using genetic approaches have uncovered the detrimental role of STING in the pathogenesis of certain monogenic autoinflammatory disorders ^{20 36}. Surprisingly, the inventors of the present application have shown that anti-STING inhibitors can be useful for the treatment of interferonopathies such as, e.g. AGS and SAVI.

The compounds of the invention are preferably compounds of formula (I): 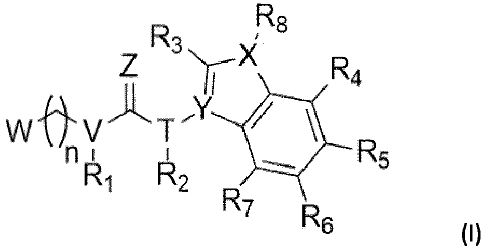 racemates, enantiomers, diastereomers and pharmaceutically acceptable salts thereof, wherein
R¹ and R² are each independently selected from H, C₁-C₁₀ alkyl, C₂-C₆ alkenyl, C₁-C₁₀ haloalkyl or a lone pair of electrons;
R³ is H;
R⁴, R⁵, R⁶ and R⁷ are each independently selected from H, C₁-C₁₀ alkyl, C₂-C₁₀ alkenyl, C₂-C₁₀ alkynyl, C₁-C₁₀ haloalkyl, C₁-C₁₀ alkoxy, C₁-C₁₀ carboxyalkyl, carboxyl, halogen, nitro, cyano, amino, amido, or ether groups;
R⁸ is selected from H, C₁-C₆ alkyl, C₂-C₆ alkenyl, C₂-C₁₀ alkynyl or C₁-C₆ haloalkyl;
X is selected from O, S, or NH;
Z is selected from O, S, or N;
Y is C, O, S or N;
T and V are independently selected from O, S, or N;
W is selected from saturated or unsaturated C₁-C₂₀ alkyl group, C₆-C₃₁ aryl group, C₆-C₃₁ heteroaryl group, C₅-C₃₁ aryloxy group, or C₅-C₃₁ aralkyl group, wherein each group is optionally substituted with 1, 2, 3 substituents independently selected from -O-CF₃, -CF₃, -O-CHF₂, halogen, -OH, -NH₂, -CN, -OCH₃, C₁-C₆ alkyl, allyl, -C(O)OR¹, -C(O)NH₂, propargyl, -NO₂, -S(O)₂NH₂, and -S(O)CH₃; and n is an integer between 0-2, with the proviso that 1-(4-ethylphenyl)-3-(1H-indol-3-yl)urea is excluded. Preferably, each group is optionally substituted with 1, 2, 3 substituents independently selected from -O-CF₃, -O-CHF₂, halogen, - OH, -NH₂, -CN, -OCH₃, C₁-C₃ alkyl, C₅-C₆ alkyl, allyl, -C(O)OR¹, -C(O)NH₂, propargyl, - NO₂, -S(O)₂NH₂, and -S(O)CH₃.
Preferably, the C₆-C₃₁ aryl group of W is substituted with C₃-C₆ alkyl or -CF₃.
Preferably, the compounds of formula I of the invention will have the following substituents,
i) R³, R⁴, R⁵, R⁶ and R⁷ are H; or
ii) Z is O; or
iii) W is a C₆-C₂₀ alkyl group or a C₆-C₃₁ aryl group, preferably a C₆-C₁₂ aryl, most preferably a C₆ aryl, optionally substituted with -CF₃, halogen (*i.e.* F, Cl, Br and I)), C₁-C₃ alkyl or C₅-C₆ alkyl; or
iv) V is N or O;
v) X is N and R⁸ is H,;
or any combinations between i), ii), iii), iv) and v).

More preferably, the compound of formula I of the invention is selected from the group consisting in 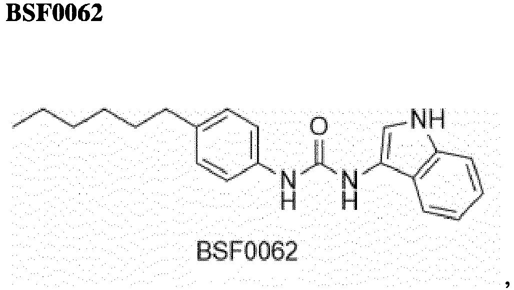 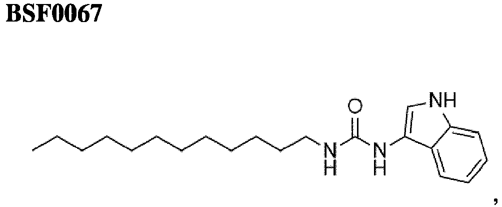 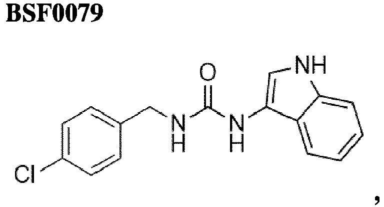 and

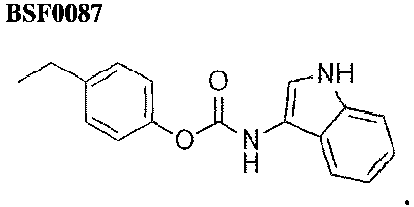

The present invention also concerns a compound of the invention having formula I, 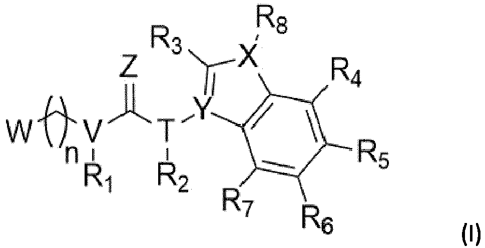 racemates, enantiomers, diastereomers and pharmaceutically acceptable salts thereof, wherein
R¹ and R² are each independently selected from H, C₁-C₁₀ alkyl, C₂-C₆ alkenyl, or C₁-C₁₀ haloalkyl or a lone pair of electrons;
R³ is H;
R⁴, R⁵, R⁶ and R⁷ are each independently selected from H, C₁-C₁₀ alkyl, C₂-C₁₀ alkenyl, C₂-C₁₀ alkynyl, C₁-C₁₀ haloalkyl, C₁-C₁₀ alkoxy, C₁-C₁₀ carboxyalkyl, carboxyl, halogen, nitro, cyano, amino, amido, or ether groups;
R⁸ is selected from H, C₁-C₆ alkyl, C₂-C₆ alkenyl, C₂-C₁₀ alkynyl or C₁-C₆ haloalkyl;
X is selected from O, S, or NH;
Z is selected from O, S, or N;
Y is C, O, S or N;
T and V are independently selected from O, S, or N;
W is selected from saturated or unsaturated C₁-C₂₀ alkyl group, C₆-C₃₁ aryl group, C₆-C₃₁ heteroaryl group, C₅-C₃₁ aryloxy group, or C₅-C₃₁ aralkyl group, wherein each group is optionally substituted with 1, 2, 3 substituents independently selected from -O-CF₃, -CF₃, -O-CHF₂, halogen, -OH, -NH₂, -CN, -OCH₃, C₁-C₆ alkyl, allyl, -C(O)OR¹, -C(O)NH₂, propargyl, -NO₂, -S(O)₂NH₂, and -S(O)CH₃; and n is an integer between 0-2, or a racemate, enantiomer, diastereomer and pharmaceutically acceptable salt thereof, for use in the treatment of a STING associated disease. Preferably, the 6-31 membered aryl group of W is substituted with C₂-C₆ alkyl or -CF₃, more preferably with C₃-C₆ alkyl.

Also envisioned is a pharmaceutical composition comprising a compound of the invention having formula I, 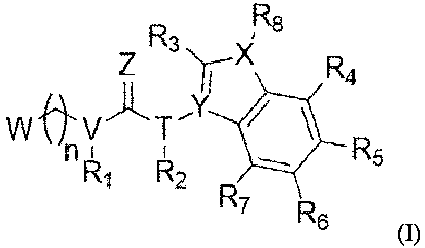 racemates, enantiomers, diastereomers and pharmaceutically acceptable salts thereof, wherein
R¹ and R² are each independently selected from H, C₁-C₁₀ alkyl, C₂-C₆ alkenyl, C₁-C₁₀ haloalkyl or a lone pair of electrons;
R³ is H;
R⁴, R⁵ , R⁶ and R⁷ are each independently selected from H, C₁-C₁₀ alkyl, C₂-C₁₀ alkenyl, C₂-C₁₀ alkynyl, C₁-C₁₀ haloalkyl, C₁-C₁₀ alkoxy, C₁-C₁₀ carboxyalkyl, carboxyl, halogen, nitro, cyano, amino, amido, or ether groups;
R⁸ is selected from H, C₁-C₆ alkyl, C₂-C₆ alkenyl, C₂-C₁₀ alkynyl or C₁-C₆ haloalkyl;
X is selected from O, S, or NH;
Z is selected from O, S, or N;
Y is C, O, S or N;
T and V are independently selected from O, S, or N;
W is selected from saturated or unsaturated C₁-C₂₀ alkyl group, C₆-C₃₁ aryl group, C₆-C₃₁ heteroaryl group, C₅-C₃₁ aryloxy group, or C₅-C₃₁ aralkyl group, wherein each group is optionally substituted with 1, 2, 3 substituents independently selected from -O-CF₃, -CF₃, -O-CHF₂, halogen, -OH, -NH₂, -CN, -OCH₃, C₁-C₆ alkyl, allyl, -C(O)OR¹, -C(O)NH₂, propargyl, -NO₂, -S(O)₂NH₂, and -S(O)CH₃; and n is an integer between 0-2 or a pharmaceutically acceptable salt thereof, and a pharmaceutically acceptable excipient. Preferably, the 6-31 membered aryl group of W is substituted with C₂-C₆ alkyl or -CF₃, more preferably with C3-C₆ alkyl.

The present invention also considers the use of a compound of the invention having formula I 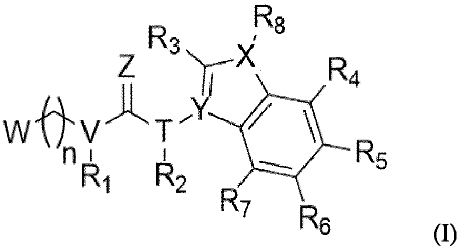 racemates, enantiomers, diastereomers and pharmaceutically acceptable salts thereof, wherein
R¹ and R² are each independently selected from H, C₁-C₁₀ alkyl, C₂-C₆ alkenyl, C₁-C₁₀ haloalkyl or a lone pair of electrons;
R³ is H;
R⁴, R⁵ , R⁶ and R⁷ are each independently selected from H, C₁-C₁₀ alkyl, C₂-C₁₀ alkenyl, C₂-C₁₀ alkynyl, C₁-C₁₀ haloalkyl, C₁-C₁₀ alkoxy, C₁-C₁₀ carboxyalkyl, carboxyl, halogen, nitro, cyano, amino, amido, or ether groups;
R⁸ is selected from H, C₁-C₆ alkyl, C₂-C₆ alkenyl, C₂-C₁₀ alkynyl or C₁-C₆ haloalkyl; X is selected from O, S, or NH;
Z is selected from O, S, or N;
Y is C, O, S or N;
T and V are independently selected from O, S, or N;
W is selected from saturated or unsaturated C₁-C₂₀ alkyl group, C₆-C₃₁ aryl group, C₆-C₃₁ heteroaryl group, C₅-C₃₁ aryloxy group, or C₅-C₃₁ aralkyl group, wherein each group is optionally substituted with 1, 2, 3 substituents independently selected from -O-CF₃, -CF₃, -O-CHF₂, halogen, -OH, -NH₂, -CN, -OCH₃, C₁-C₆ alkyl, allyl, -C(O)OR¹, -C(O)NH₂, propargyl, -NO₂, -S(O)₂NH₂, and -S(O)CH₃; and n is an integer between 0-2 or a pharmaceutically acceptable salt thereof, and a pharmaceutically acceptable excipient, or pharmaceutical composition of the invention, in the manufacture of a medicament. Preferably, the medicament is for the treatment of a STING associated disease.

The present invention also considers a compound that inhibits the signaling molecule STING by binding at least one amino acid of a region including the amino acid residues 85 to 95. Preferably, the binding is a covalent binding. Most preferably, the compound of the invention inhibits the signaling molecule STING by covalently binding amino acid residue 91 (CYS91).

A kit comprising (1) a composition comprising a pharmaceutically effective amount of invention having formula I 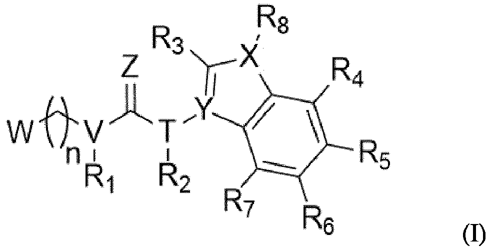 racemates, enantiomers, diastereomers and pharmaceutically acceptable salts thereof, wherein
R¹ and R² are each independently selected from H, C₁-C₁₀ alkyl, C₂-C₆ alkenyl, C₁-C₁₀ haloalkyl or a lone pair of electrons;
R³ is H;
R⁴, R⁵, R⁶ and R⁷ are each independently selected from H, C₁-C₁₀ alkyl, C₂-C₁₀ alkenyl, C₂-C₁₀ alkynyl, C₁-C₁₀ haloalkyl, C₁-C₁₀ alkoxy, C₁-C₁₀ carboxyalkyl, carboxyl, halogen, nitro, cyano, amino, amido, or ether groups;
R⁸ is selected from H, C₁-C₆ alkyl, C₂-C₆ alkenyl, C₂-C₁₀ alkynyl or C₁-C₆ haloalkyl;
X is selected from O, S, or NH;
Z is selected from O, S, or N;
Y is C, O, S or N;
T and V are independently selected from O, S, or N;
W is selected from saturated or unsaturated C₁-C₂₀ alkyl group, C₆-C₃₁ aryl group, C6-C₃₁ heteroaryl group, C₅-C₃₁ aryloxy group, or C₅-C₃₁ aralkyl group, wherein each group is optionally substituted with 1, 2, 3 substituents independently selected from -O-CF₃, -CF₃, -O-CHF₂, halogen (*i.e.* F, Cl, Br and I), -OH, -NH₂, -CN, -OCH₃, C₁-C₆ alkyl, allyl, -C(O)OR¹, - C(O)NH₂, propargyl, -NO₂, -S(O)₂NH₂, and -S(O)CH₃; and n is an integer between 0-2 or a pharmaceutically acceptable salt thereof, and a pharmaceutically acceptable excipient.or racemates, enantiomers, diastereomers and pharmaceutically acceptable salts thereof; and (2) instructions for use is also envisioned.

The term "alkyl" refers to monovalent straight-chained and branched alkyl groups, saturated or unsaturated, having 1 to 20 carbon atoms, such as C1-C3 alkyl, C1-C4 alkyl, Cl-C6 alkyl or C1-C12 alkyl. Examples of straight chain alkyl groups include, but are not limited to, those with from 1 to 20 carbon atoms, preferably 1 to 12 carbon atoms such as methyl, ethyl, n-propyl, n-butyl, n-pentyl, n-hexyl groups, n-heptyl, n-octyl, n-nonyl and n-decyl groups. Examples of branched alkyl groups include, but are not limited to, isopropyl, iso-butyl, sec-butyl, tert-butyl, isopentyl, and 2,2-dimethylpropyl groups. Alkyl groups may be substituted or unsubstituted. Representative substituted alkyl groups may be substituted one or more times and are preferably selected from the non-limiting group comprising -O-CF₃, -CF₃, -O-CHF₂, halogen, -OH, -NH₂, -CN, -OCH₃, C₁-C₆ alkyl, allyl, -C(O)OR¹, - C(O)NH₂, propargyl, -NO₂, -S(O)₂NH₂, and -S(O)CH₃.

The term "alkenyl" refers to a straight or branched hydrocarbon containing one or more double bonds and having, preferably, 2 to 6 carbon atoms. Examples of alkenyl comprise ethenyl, propenyl, allyl, and 1,4-butadienyl.

The term "haloalkyl" refers to any alkyl radical having one or more hydrogen atoms replaced by a halogen atom (i.e. F, Cl, Br and I) and having, preferably, 2 to 10 carbon atoms.

### Examples of alkenyl comprise

The term "alkynyl" refers to any of a series of aliphatic hydrocarbons with a carbon-carbon triple bond and the general formula CnH2n-2 and having, preferably, 2 to 10 carbon atoms.

The term "alkoxy" refers to an alkyl group singularly bonded to oxygen and having, preferably, 2 to 10 carbon atoms.

The term "carboxyalkyl" refers to a group comprising an alkyl and a carboxy group and having, preferably, 1 to 10 carbon atoms.

The term "aryl" refers to cyclic aromatic hydrocarbons that do not contain heteroatoms. Aryl groups include monocyclic, bicyclic and polycyclic ring systems. Thus, aryl groups include, but are not limited to C₆-C₃₁ aryl, e.g. C6 aryl, (such as phenyl, benzyl, tolyl, xylyl, benzyliden, benzoyl), C₆-C₁₈ aryl, and C₆-C₁₀ aryl (such as azulenyl, heptalenyl, biphenylenyl, indacenyl, fluorenyl, phenanthrenyl, triphenylenyl, pyrenyl, naphthacenyl, chrysenyl, biphenyl, anthracenyl, indenyl, indanyl, pentalenyl, naphthyl groups). The term "aryl" also includes groups containing fused rings, such as fused aromatic-aliphatic ring systems (such as naphtyl, indanyl, tetrahydronaphthyl, and the like). Groups such as phenyl and naphtyl are preferred. Aryl groups may be substituted or unsubstituted. Representative substituted aryl groups may be mono-substituted or substituted more than once and are preferably selected from the non-limiting group comprising -O-CF₃, -CF₃, -O-CHF₂, halogen, -OH, -NH₂, -CN, -OCH₃, C₁-C₆ alkyl, allyl, -C(O)OR¹, -C(O)NH₂, propargyl, -NO₂, - S(O)₂NH₂, and -S(O)CH₃.

"Heteroaryl" refers to mono or multicyclic aryl group in which one or more of the aromatic carbon atoms (and any associated hydrogen atoms) are independently replaced with the same or different heteroatom (such as, e.g., N, O and S) or heteroatomic group. Multicyclic ring systems are included in heteroaryl and may be attached at the ring with the heteroatom or the aryl ring. Heteroaryl groups include, but are not limited to, groups derived from acridine, benzoimidazole, benzothiophene, benzofuran, benzoxazole, benzothiazole, carbazole, carboline, cinnoline, furan, imidazole, imidazopyridine, indazole, indole, indoline, indolizine, isobenzofuran, isochromene, isoindole, isoindoline, isoquinoline, isothiazole, isoxazole, naphthyridine, oxadiazole, oxazole, perimidine, phenanthridine, phenanthroline, phenazine, phthalazine, pteridine, purine, pyran, pyrazine, pyrazole, pyridazine, pyridine, pyridone, pyrimidine, pyrrole, pyrrolizine, quinazoline, quinoline, quinolizine, quinoxaline, tetrazole, thiadiazole, thiazole, thiophene, triazole, xanthene, and the like. Heteroaryl groups may have 6-31 members, 6-20 members, 6-15 members, 6-12 members, 5-10 members, or 5-6 members.

"Aralkyl" (also "Arylalkyl") refers to any combination aryl group and an alkyl group. Arylalkyl groups include, but are not limited to, those groups derived from benzyl, tolyl, dimethylphenyl, 2-phenylethan-1-yl, 2-naphthylmethyl, and the like. An arylalkyl group comprises from 6 to 30 carbon atoms, for example the alkyl group can comprise from 1 to 10 carbon atoms and the aryl group can comprise from 5 to 20 carbon atoms.

The term "comprise/comprising" is generally used in the sense of include/including, that is to say permitting the presence of one or more features or components.

As used in the specification and claims, the singular form "a", "an" and "the" include plural references unless the context clearly dictates otherwise.

As used herein the terms " subject", or " patient" are well-recognized in the art, and, are used interchangeably herein to refer to a mammal, including dog, cat, rat, mouse, monkey, cow, horse, goat, sheep, pig, camel, and, most preferably, a human. In some embodiments, the subject is a subject in need of treatment or a subject suffering from a STING associated disease. However, in other aspects, the subject can be a normal subject. The term does not denote a particular age or sex. Thus, adult and newborn subjects, whether male or female, are intended to be covered.

Reference throughout this specification to "one aspect" "an aspect" "another aspect," "a particular aspect," combinations thereof means that a particular feature, structure or characteristic described in connection with the invention aspect is included in at least one aspect of the present invention. Furthermore, the particular features, structures, or characteristics may be combined in any suitable manner in one or more aspects.

The term "treatment" or "treating" means any administration of a composition, pharmaceutical composition, compound, etc... of the disclosure to a subject for the purpose of:
(i) inhibiting the disease, condition, or disorder, that is, arresting the development of clinical symptoms; and/or
(ii) relieving the disease, condition, or disorder, that is, causing the regression of clinical symptoms.

The term "prevention" or "preventing" means any administration of a composition, pharmaceutical composition, compound, etc... of the disclosure to a subject for the purpose of preventing the disease, condition, or disorder, that is, causing the clinical symptoms of the disease.

In the context of the present invention, the disease, condition, or disorder is a STING associated disease, condition, or disorder that is alleviated by treatment with a compound, composition, or pharmaceutical composition, of the disclosure that inhibits (e.g. antagonizes) the signaling molecule STING.

In some aspects, the condition, disease or disorder is cancer. Non-limiting examples of cancer include melanoma, carcinoma, lymphoma, blastoma, sarcoma, and leukemia or lymphoid malignancies. More particular examples of such cancers include breast cancer, colon cancer, rectal cancer, colorectal cancer, kidney or renal cancer, clear cell cancer lung cancer including small-cell lung cancer, non- small cell lung cancer, adenocarcinoma of the lung and squamous carcinoma of the lung, squamous cell cancer (e.g. epithelial squamous cell cancer), cervical cancer, ovarian cancer, prostate cancer, prostatic neoplasms, liver cancer, bladder cancer, cancer of the peritoneum, hepatocellular cancer, gastric or stomach cancer including gastrointestinal cancer, gastrointestinal stromal tumor, pancreatic cancer, head and neck cancer, glioblastoma, retinoblastoma, astrocytoma, thecomas, arrhenoblastomas, hepatoma, hematologic malignancies including non-Hodgkins lymphoma (NHL), multiple myeloma, myelodysplasia disorders, myeloproliferative disorders, chronic myelogenous leukemia, and acute hematologic malignancies, endometrial or uterine carcinoma, endometriosis, endometrial stromal sarcoma, fibrosarcomas, choriocarcinoma, salivary gland carcinoma, vulval cancer, thyroid cancer, esophageal carcinomas, hepatic carcinoma, anal carcinoma, penile carcinoma, nasopharyngeal carcinoma, laryngeal carcinomas, Kaposi's sarcoma, mast cell sarcoma, ovarian sarcoma, uterine sarcoma, melanoma, malignant mesothelioma, skin carcinomas, Schwannoma, oligodendroglioma, neuroblastomas, neuroectodermal tumor, rhabdomyosarcoma, osteogenic sarcoma, leiomyosarcomas, Ewing Sarcoma, peripheral primitive neuroectodermal tumor, urinary tract carcinomas, thyroid carcinomas, Wilm's tumor, as well as abnormal vascular proliferation associated with phakomatoses, edema (such as that associated with brain tumors), and Meigs' syndrome. In some cases, the cancer is melanoma.

In some aspects, the condition, disease or disorder is a neurological disorder, which includes disorders that involve the central nervous system (brain, brainstem and cerebellum), the peripheral nervous system (including cranial nerves), and the autonomic nervous system (parts of which are located in both central and peripheral nervous system). Non-limiting examples of neurological disorder include acquired epileptiform aphasia; acute disseminated encephalomyelitis; adrenoleukodystrophy; age-related macular degeneration; agenesis of the corpus callosum; agnosia; Aicardi syndrome; Alexander disease; Alpers' disease; alternating hemiplegia; Alzheimer's disease; Vascular dementia; amyotrophic lateral sclerosis; anencephaly; Angelman syndrome; angiomatosis; anoxia; aphasia; apraxia; arachnoid cysts; arachnoiditis; Anronl-Chiari malformation; arteriovenous malformation; Asperger syndrome; ataxia telegiectasia; attention deficit hyperactivity disorder; autism; autonomic dysfunction; back pain; Batten disease; Behcet's disease; Bell's palsy; benign essential blepharospasm; benign focal; amyotrophy; benign intracranial hypertension; Binswanger's disease; blepharospasm; Bloch Sulzberger syndrome; brachial plexus injury; brain abscess; brain injury; brain tumors (including glioblastoma multiforme); spinal tumor; Brown-Sequard syndrome; Canavan disease; carpal tunnel syndrome; causalgia; central pain syndrome; central pontine myelinolysis; cephalic disorder; cerebral aneurysm; cerebral arteriosclerosis; cerebral atrophy; cerebral gigantism; cerebral palsy; Charcot-Marie-Tooth disease; chemotherapy-induced neuropathy and neuropathic pain; Chiari malformation; chorea; chronic inflammatory demyelinating polyneuropathy; chronic pain; chronic regional pain syndrome; Coffin Lowry syndrome; coma, including persistent vegetative state; congenital facial diplegia; corticobasal degeneration; cranial arteritis; craniosynostosis; Creutzfeldt-Jakob disease; cumulative trauma disorders; Cushing's syndrome; cytomegalic inclusion body disease; cytomegalovirus infection; dancing eyes-dancing feet syndrome; Dandy-Walker syndrome; Dawson disease; De Morsier's syndrome; Dejerine-Klumke palsy; dementia; dermatomyositis; diabetic neuropathy; diffuse sclerosis; dysautonomia; dysgraphia; dyslexia; dystonias; early infantile epileptic encephalopathy; empty sella syndrome; encephalitis; encephaloceles; encephalotrigeminal angiomatosis; epilepsy; Erb's palsy; essential tremor; Fabry's disease; Fahr's syndrome; fainting; familial spastic paralysis; febrile seizures; Fisher syndrome; Friedreich's ataxia; fronto-temporal dementia and other "tauopathies"; Gaucher's disease; Gerstmann's syndrome; giant cell arteritis; giant cell inclusion disease; globoid cell leukodystrophy; Guillain-Barre syndrome; HTLV-1 -associated myelopathy; Hallervorden-Spatz disease; head injury; headache; hemifacial spasm; hereditary spastic paraplegia; heredopathia atactica polyneuritiformis; herpes zoster oticus; herpes zoster; Hirayama syndrome; HIV-associated dementia and neuropathy (also neurological manifestations of AIDS); holoprosencephaly; Huntington's disease and other polyglutamine repeat diseases; hydranencephaly; hydrocephalus; hypercortisolism; hypoxia; immune-mediated encephalomyelitis; inclusion body myositis; incontinentia pigmenti; infantile phytanic acid storage disease; infantile refsum disease; infantile spasms; inflammatory myopathy; intracranial cyst; intracranial hypertension; Joubert syndrome; Kearns-Sayre syndrome; Kennedy disease Kinsbourne syndrome; Klippel Feil syndrome; Krabbe disease; Kugelberg-Welander disease; kuru; Lafora disease; Lambert-Eaton myasthenic syndrome; Landau-Kleffner syndrome; lateral medullary (Wallenberg) syndrome; learning disabilities; Leigh's disease; Lennox-Gustaut syndrome; Lesch-Nyhan syndrome; leukodystrophy; Lewy body dementia; Lissencephaly; locked-in syndrome; Lou Gehrig's disease (i.e., motor neuron disease or amyotrophic lateral sclerosis); lumbar disc disease; Lyme disease-neurological sequelae; Machado-Joseph disease; macrencephaly; megalencephaly; Melkersson-Rosenthal syndrome; Menieres disease; meningitis; Menkes disease; metachromatic leukodystrophy; microcephaly; migraine; Miller Fisher syndrome; mini-strokes; mitochondrial myopathies; Mobius syndrome; monomelic amyotrophy; motor neuron disease; Moyamoya disease; mucopolysaccharidoses; milti-infarct dementia; multifocal motor neuropathy; multiple sclerosis and other demyelinating disorders; multiple system atrophy with postural hypotension; p muscular dystrophy; myasthenia gravis; myelinoclastic diffuse sclerosis; myoclonic encephalopathy of infants; myoclonus; myopathy; myotonia congenital; narcolepsy; neurofibromatosis; neuroleptic malignant syndrome; neurological manifestations of AIDS; neurological sequelae of lupus; neuromyotonia; neuronal ceroid lipofuscinosis; neuronal migration disorders; Niemann-Pick disease; O'Sullivan-McLeod syndrome; occipital neuralgia; occult spinal dysraphism sequence; Ohtahara syndrome; olivopontocerebellar atrophy; opsoclonus myoclonus; optic neuritis; orthostatic hypotension; overuse syndrome; paresthesia; Parkinson's disease; paramyotonia congenital; paraneoplastic diseases; paroxysmal attacks; Parry Romberg syndrome; Pelizaeus-Merzbacher disease; periodic paralyses; peripheral neuropathy; painful neuropathy and neuropathic pain; persistent vegetative state; pervasive developmental disorders; photic sneeze reflex; phytanic acid storage disease; Pick's disease; pinched nerve; pituitary tumors; polymyositis; porencephaly; post-polio syndrome; postherpetic neuralgia; postinfectious encephalomyelitis; postural hypotension; Prader-Willi syndrome; primary lateral sclerosis; prion diseases; progressive hemifacial atrophy; progressive multifocal leukoencephalopathy; progressive sclerosing poliodystrophy; progressive supranuclear palsy; pseudotumor cerebri; Ramsay-Hunt syndrome (types I and II); Rasmussen's encephalitis; reflex sympathetic dystrophy syndrome; Refsum disease; repetitive motion disorders; repetitive stress injuries; restless legs syndrome; retrovirus-associated myelopathy; Rett syndrome; Reye's syndrome; Saint Vitus dance; Sandhoff disease; Schilder's disease; schizencephaly; septo-optic dysplasia; shaken baby syndrome; shingles; Shy-Drager syndrome; Sjögren's syndrome; sleep apnea; Soto's syndrome; spasticity; spina bifida; spinal cord injury; spinal cord tumors; spinal muscular atrophy; Stiff-Person syndrome; stroke; Sturge-Weber syndrome; subacute sclerosing panencephalitis; subcortical arteriosclerotic encephalopathy; Sydenham chorea; syncope; syringomyelia; tardive dyskinesia; Tay-Sachs disease; temporal arteritis; tethered spinal cord syndrome; Thomsen disease; thoracic outlet syndrome; Tic Douloureux; Todd's paralysis; Tourette syndrome; transient ischemic attack; transmissible spongiform encephalopathies; transverse myelitis; traumatic brain injury; tremor; trigeminal neuralgia; tropical spastic paraparesis; tuberous sclerosis; vascular dementia (multi-infarct dementia); vasculitis including temporal arteritis; Von Hippel-Lindau disease; Wallenberg's syndrome; Werdnig-Hoffman disease; West syndrome; whiplash; Williams syndrome; Wildon's disease; amyotrophe lateral sclerosis and Zellweger syndrome.

In some aspects, the condition, disease or disorder is an autoimmune diseases, e.g. a cytosolic DNA-triggered autoinflammatory disease. Non-limiting examples include rheumatoid arthritis, systemic lupus erythematosus, multiple sclerosis, inflammatory bowel diseases (IBDs) comprising Crohn disease (CD) and ulcerative colitis (UC), which are chronic inflammatory conditions with polygenic susceptibility. In certain aspects, the condition is an inflammatory bowel disease. In certain aspects, the condition is Crohn's disease, autoimmune colitis, iatrogenic autoimmune colitis, ulcerative colitis, colitis induced by one or more chemotherapeutic agents, colitis induced by treatment with adoptive cell therapy, colitis associated by one or more alloimmune diseases (such as graft-vs-host disease, e.g., acute graft vs. host disease and chronic graft vs. host disease), radiation enteritis, collagenous colitis, lymphocytic colitis, microscopic colitis, and radiation enteritis. In certain of these aspects, the condition is alloimmune disease (such as graft-vs-host disease, e.g., acute graft vs. host disease and chronic graft vs. host disease), celiac disease, irritable bowel syndrome, rheumatoid arthritis, lupus, scleroderma, psoriasis, cutaneous T-cell lymphoma, uveitis, and mucositis (e.g., oral mucositis, esophageal mucositis or intestinal mucositis).

In some aspects, modulation of the immune system by STING provides for the treatment of diseases, including diseases caused by foreign agents. Exemplary infections by foreign agents which may be treated and/or prevented by the method of the present invention include an infection by a bacterium (e.g., a Gram-positive or Gram-negative bacterium), an infection by a fungus, an infection by a parasite, and an infection by a virus. In one aspect of the present invention, the infection is a bacterial infection (e.g., infection by E. coli, Klebsiella pneumoniae, Pseudomonas aeruginosa, Salmonella spp., Staphylococcus aureus, Streptococcus spp., or vancomycin-resistant enterococcus) or sepsis. In another aspect, the infection is a fungal infection (e.g. infection by a mould, a yeast, or a higher fungus). In still another embodiment, the infection is a parasitic infection (e.g., infection by a single-celled or multicellular parasite, including Giardia duodenalis, Cryptosporidium parvum, Cyclospora cayetanensis, and Toxoplasma gondiz). In yet another aspect, the infection is a viral infection (e.g., infection by a virus associated with AIDS, avian flu, chickenpox, cold sores, common cold, gastroenteritis, glandular fever, influenza, measles, mumps, pharyngitis, pneumonia, rubella, SARS, and lower or upper respiratory tract infection (e.g., respiratory syncytial virus)).

In some aspects, the condition, disease or disorder is selected from the non-limiting group comprising hepatitis B, cardiovascular diseases (including e.g. myocardial infarction) and age-related macular degeneration.

Preferably, the condition, disease or disorder is selected from the group comprising a cancer, a neurological disorder, an autoimmune disease, hepatitis B, uveitis, cardiovascular disease, age-related macular degeneration and mucositis.

The term "pharmaceutical" as used herein refers to a chemical substance intended for use in the cure, treatment, or prevention of disease. Details on techniques for formulation and administration of such compositions may be found in Remington, The Science and Practice of Pharmacy 21st Edition (Mack Publishing Co., Easton, Pa.) and Nielloud and Marti-Mestres, Pharmaceutical Emulsions and Suspensions: 2nd Edition (Marcel Dekker, Inc, New York).
For the purposes of this disclosure, the pharmaceutical compositions may be administered by a variety of means including orally, parenterally, by inhalation spray, topically, or rectally in formulations containing pharmaceutically acceptable carriers, adjuvants and vehicles. The term parenteral as used here includes but is not limited to subcutaneous, intravenous, intramuscular, intraarterial, intradermal, intrathecal and epidural injections with a variety of infusion techniques. Intraarterial and intravenous injection as used herein includes administration through catheters. Administration via intracoronary stents and intracoronary reservoirs is also contemplated. The term oral as used herein includes, but is not limited to oral ingestion, or delivery by a sublingual or buccal route. Oral administration includes fluid drinks, energy bars, as well as pill formulations

Pharmaceutical compositions may be in any form suitable for the intended method of administration. When used for oral use for example, tablets, troches, lozenges, aqueous or oil suspensions, dispersible powders or granules, emulsions, hard or soft capsules, syrups or elixirs may be prepared. Compositions intended for oral use may be prepared according to any method known to the art for the manufacture of pharmaceutical compositions and such compositions may contain one or more agents including sweetening agents, flavoring agents, coloring agents and preserving agents, in order to provide a palatable preparation. Tablets containing a drug compound in admixture with non-toxic pharmaceutically acceptable excipient which are suitable for manufacture of tablets are acceptable. These excipients may be, for example, inert diluents, such as calcium or sodium carbonate, lactose, calcium or sodium phosphate; granulating and disintegrating agents, such as maize starch, or alginic acid; binding agents, such as starch, gelatin or acacia; and lubricating agents; such as magnesium stearate, stearic acid or talc. Tablets may be uncoated, or may be coated by known techniques including enteric coating, colonic coating, or microencapsulation to delay disintegration and adsorption in the gastrointestinal tract and/or provide a sustained action over a longer period. For example, a time delay material such as glyceryl monostearate or glyceryl distearate alone or with a wax may be employed.

Formulations for oral use may be also presented as hard gelatin capsules where the drug compound is mixed with an inert solid diluent, for example calcium phosphate or kaolin, or as soft gelatin capsules wherein the active ingredient is mixed with water or an oil medium, such as peanut oil, liquid paraffin or olive oil.

Such excipients include a suspending agent, such as sodium carboxymethylcellulose, methylcellulose, hydroxypropyl methylcellulose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycetanol), a condensation product of ethylene oxide with a partial ester derived from a fatty acid and a hexitol anhydride (e.g., polyoxyethylene sorbitan monooleate). The aqueous suspension may also contain one or more preservatives such as ethyl or n-propyl p-hydroxy-benzoate, one or more coloring agents, one or more flavoring agents and one or more sweetening agents, such as sucrose or saccharin.

Oil suspensions may be formulated by suspending the active ingredient in a vegetable oil, such as arachis oil, olive oil, sesame oil or coconut oil, or a mineral oil such as liquid paraffin. The oral suspensions may contain a thickening agent, such as beeswax, hard paraffin or cetyl alcohol. Sweetening agents, such as those set forth above, and flavoring agents may be added to provide a palatable oral preparation. These compositions may be preserved by the addition of an antioxidant such as ascorbic acid.

Dispersible powders and granules of the disclosure suitable for preparation of an aqueous suspension by the addition of water provide the active ingredient in admixture with a dispersing or wetting agent, a suspending agent, and one or more preservatives. Suitable dispersing or wetting agents and suspending agents are exemplified by those disclosed above. Additional excipients, for example sweetening, flavoring and coloring agents, may also be present.

The pharmaceutical compositions of the disclosure may also be in the form of oil-in-water emulsions. The oily phase may be a vegetable oil, such as olive oil or arachis oil, a mineral oil, such as liquid paraffin, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan monooleate, and condensation products of these partial esters with ethylene oxide, such as polyoxyethylene sorbitan monooleate. The emulsion may also contain sweetening and flavoring agents.

Syrups and elixirs may be formulated with sweetening agents, such as glycerol, sorbitol or sucrose. Such formulations may also contain a demulcent, a preservative, a flavoring or a coloring agent.

The pharmaceutical compositions of the disclosure may be in the form of a sterile injectable preparation, such as a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent such as a solution in 1,3-butane-diol or prepared as a lyophilized powder. Among the acceptable vehicles and solvents that may be employed are water, Ringer's solution and isotonic sodium chloride solution. In addition, sterile fixed oils may conventionally be employed as a solvent or suspending medium. For this purpose any bland fixed oil may be employed including synthetic mono- or diglycerides. In addition, fatty acids such as oleic acid may likewise be used in the preparation of injectables.

The amount of active ingredient, e.g. the compounds of formula I, which may be combined with the carrier material to produce a single dosage form will vary depending upon the host treated and the particular mode of administration. For example, a time-release formulation intended for oral administration to humans may contain approximately 20 to 500 mg of active material compounded with an appropriate and convenient amount of carrier material which may vary from about 5 to about 95% of the total compositions. It is preferred that the pharmaceutical composition be prepared which provides easily measurable amounts for administration. Typically, an effective amount to be administered systemically is about 0.1 mg/kg to about 100 mg/kg and depends upon a number of factors including, for example, the age and weight of the subject (e.g., a mammal such as a human), the precise condition requiring treatment and its severity, the route of administration, and will ultimately be at the discretion of the attendant physician or veterinarian. It will be understood, however, that the specific dose level for any particular patient will depend on a variety of factors including the activity of the specific compound employed, the age, body weight, general health, sex and diet of the individual being treated; the time and route of administration; the rate of excretion; other drugs which have previously been administered; and the severity of the particular condition undergoing therapy, as is well understood by those skilled in the art.

The present application also provides pharmaceutically acceptable salts, hydrates, solvates, isomers, tautomers, stereoisomers, enantiomers, racemates, atropisomers, polymorphs, prodrugs, or a mixture thereof, of the compounds described herein.

As used herein, pharmaceutically acceptable salts include, but are not limited, acetates, ascorbates, benzenesulphonates, benzoates, besylates, bicarbonates, bitartrates, bromides/hydrobromides, Ca-edetates/edetates, camsylates, carbonates, chlorides/hydrochlorides, citrates, edisylates, ethane disulphonates, estolates esylates, fumarates, gluceptates, gluconates, glutamates, glycolates, glycollylarsnilates, hexylresorcinates, hydrabamines, hydroxymaleates, hydroxynaphthoates, iodides, isothionates, lactates, lactobionates, malates, maleates, mandelates, methanesulphonates, mesylates, methylbromides, methylnitrates, methylsulphates, mucates, napsylates, nitrates, oxalates, pamoates, pantothenates, phenyl acetates, phosphates/diphosphates, polygalacturonates, propionates, salicylates, stearates, subacetates, succinates, sulphamides, sulphates, tannates, tartrates, teoclates, toluenesulphonates, triethiodides, ammonium, benzathines, chloroprocaines, cholines, diethanolamines, ethylenediamines, meglumines and procaines.

Further pharmaceutically acceptable salts can be formed with cations from metals like aluminium, calcium, lithium, magnesium, potassium, sodium, zinc and the like (also see Pharmaceutical salts, Birge, S.M. et al., J. Pharm. Sci., (1977), 66, 1- 19).

"Isomers" refers to compounds that have the same molecular formula. As used herein, the term isomers include double bond isomers, racemates, stereoisomers, enantiomers, diastereomers, and atropisomers. Single isomers, such as enantiomers or diastereomers, can be obtained by asymmetric synthesis or by resolution of a mixture of isomers. Resolution of a mixture of isomers (e.g. racemates) maybe accomplished, for example, by conventional methods such as crystallization in the presence of a resolving agent, or chromatography, using, for example a chiral high pressure liquid chromatography (HPLC) column. "Double bond isomers" refer to Z- and E- forms (or cis- and trans- forms) of the compounds with carbon-carbon double bonds.

"Racemates" refers to a mixture of enantiomers, usually a 50:50 mixture of two enantiomers.

"Stereoisomers" or "stereoisomeric forms" refer to compounds that differ in the chirality of one or more stereocenters. Stereoisomers include enantiomers and diastereomers. The compounds may exist in stereoisomeric form if they possess one or more asymmetric centers or a double bond with asymmetric substitution and, therefore, can be produced as individual stereoisomers or as mixtures. Unless otherwise indicated, the description is intended to include individual stereoisomers as well as mixtures. The methods for the determination of stereochemistry and the separation of stereoisomers are well-known in the art (see, e.g., Chapter 4 of Advanced Organic Chemistry, 4th ed., J. March, John Wiley and Sons, New York, 1992). [0019] "Tautomers" or "tautomeric formers" refer to alternate forms of a compound that differ in the position of a proton, such as enol-keto and imine-enamine tautomers, or heteroaryls such as pyrazoles, imidazoles, benzimidazoles, triazoles, and tetrazoles.

A "solvate" is formed by the interaction of a solvent and a compound. Solvates of salts of the compounds of any of the formulae described herein are also provided. Hydrates of the compounds of any of the formulae are also provided. "Hydrate" refers to a complex formed by the combining a compound disclosed herein and water. The term includes stoichiometric as well as non-stoichiometric hydrates.

A "prodrug" is defined in the pharmaceutical field as a biologically inactive derivative of a drug that upon administration to the human body is converted to the biologically active parent drug according to some chemical or enzymatic pathway.

A therapeutically effective amount for a particular patient may vary depending on factors such as the condition being treated, the overall health of the patient, the route and dose of administration and the severity of side effects. Guidance for methods of treatment and diagnosis is available (see, e.g., Maynard, et al. (1996) A Handbook of SOPs for Good Clinical Practice, Interpharm Press, Boca Raton, Fla.; Dent (2001) Good Laboratory and Good Clinical Practice, Urch Publ., London, UK).

An effective amount may be given in one dose, but is not restricted to one dose. Thus, the administration can be two, three, four, five, six, seven, eight, nine, ten, eleven, twelve, thirteen, fourteen, fifteen, sixteen, seventeen, eighteen, nineteen, twenty, or more, administrations of pharmaceutical composition. Where there is more than one administration of a pharmaceutical composition in the present methods, the administrations can be spaced by time intervals of one minute, two minutes, three, four, five, six, seven, eight, nine, ten, or more minutes, by intervals of about one hour, two hours, three, four, five, six, seven, eight, nine, ten, 11, 12, 13, 14, 15, 16, 17, 18, 19, 20, 21, 22, 23, 24 hours, and so on. In the context of hours, the term "about" means plus or minus any time interval within 30 minutes. The administrations can also be spaced by time intervals of one day, two days, three days, four days, five days, six days, seven days, eight days, nine days, ten days, 11 days, 12 days, 13 days, 14 days, 15 days, 16 days, 17 days, 18 days, 19 days, 20 days, 21 days, and combinations thereof. The invention is not limited to dosing intervals that are spaced equally in time, but encompass doses at non-equal intervals.

A dosing schedule of, for example, once/week, twice/week, three times/week, four times/week, five times/week, six times/week, seven times/week, once every two weeks, once every three weeks, once every four weeks, once every five weeks, and the like, is available for the invention. The dosing schedules encompass dosing for a total period of time of, for example, one week, two weeks, three weeks, four weeks, five weeks, six weeks, two months, three months, four months, five months, six months, seven months, eight months, nine months, ten months, eleven months, and twelve months.

Co- administration is also envisioned. Methods for co-administration with an additional therapeutic agent are well known in the art (Hardman, et al. (eds.) (2001) Goodman and Gilman's The Pharmacological Basis of Therapeutics, 10th ed., McGraw-Hill, New York, N.Y.; Poole and Peterson (eds.) (2001) Pharmacotherapeutics for Advanced Practice: A Practical Approach, Lippincott, Williams & Wilkins, Phila., Pa.; Chabner and Longo (eds.) (2001) Cancer Chemotherapy and Biotherapy, Lippincott, Williams & Wilkins, Phila., Pa.)

The present invention also concerns a method of treating and/or preventing a STING associated disease comprising administering a pharmaceutical composition of the invention.

Also envisioned is a method of blocking the activation-induced palmitoylation of STING and recruitment of TBK1 by contacting a cell expressing STING with a compound of the invention.

As disclosed above, the present invention also provides a kit. The terms "kit" and "pharmaceutical kit" refer to a commercial kit or package comprising, in one or more suitable containers, one or more pharmaceutical compositions of the invention, or compounds of the invention, and instructions for their use. Such kits may also be referred to by the terms "package" or "pharmaceutical package".

### EXAMPLES

### Methods

### METHODS

### Stimulation of human THP1 cells

THP1 cells were seeded at a concentration of 1 Mio/ ml in 24-well plates and treated overnight with phorbol 12-myristate 13-acetate (PMA) (day 0). On day 1, THP1 cells were pre-treated with F2740-0151 at 0.5 µM and 1 µM or DMSO, respectively. After 90 mins, THP1 cells were stimulated with cGAMP complexed with lipofectamine 2000 (Invitrogen) or triphosphate RNA complexed with lipofectamine 2000. After 3h, cell were lysed and subjected to RNA purification. RT-qPCR analysis was performed using the following primers:
hsGAPDH forward, 5•-GAGTCAACGGATTTGGTCGT-3•;
hsGAPDH reverse, 5•-GACAAGCTTCCCGTTCTCAG-3•;
hsIFNB1 forward, 5•-CAGCATCTGCTGGTTGAAGA-3•;
hsIFNB1 reverse, 5•-CATTACCTGAAGGCCAAGGA-3•.

### Cells and cell culture conditions

Cells were cultured under 5 % CO2 and 5 % 02 at 3•°C in Dulbecco's modified Eagle medium (DMEM) (Life Technologies) containing 10 % (v/v) fetal bovine serum (FBS), 1 % (v/v) penicillin (100•IU•ml•1)/streptomycin (100••g•ml•1).

### High-throughput chemical compound screen.

HEK293T cells expressing human STING were transfected using GeneJuice (Millipore) with a construct encoding for cGAS in conjunction with an IFN-• firefly luciferase reporter plasmid. 3 h after transfection cells were seeded in 384-well plates (Corning) coated with library compounds. For each compounds, a 40 nl volume was selected to obtain a concentration of 10 µM in the assay plates. The amount of DMSO in each well was normalised to 0.1 %. After overnight treatment, cells were lysed in lysis buffer (25 mM Tris-phosphate (pH 7.8), 2 mM DTT, 2 mM 1,2-diaminocyclohexane-N,N,N',N'-tetraacetic acid, 10 % glycerol, 1 % Triton® X-100) for 20 min followed by the addition of firefly luciferase. Reporter activity was measured using a Tecan Infinite plate reader. The screen was performed using on ∼ 30.000 compounds from a chemically diverse compound collection available at the BSF core facility at EPFL.

### Western blot analysis.

Cells were lysed in 1 × Laemmli buffer, immobilized protein on beads in sample reducing buffer followed by denaturing at 95 °C for 5 min. Cell lysates were separated by 12 % SDS-PAGE and transferred onto PVDF membranes. Blots were incubated with anti-STING (D2P2F), Phospho-TBKl (D52C2), TBK-1 (D1B4) (all Cell Signaling), anti-Transferrin Receptor (Thermo Scientific, 136800) or anti-Flag M2 (Sigma, F3165). As secondary antibodies, anti-rabbit-IgG-HRP or anti-mouse-IgG-HRP (1:2000 dilution) (Santa Cruz Biotechnology) were used. Anti-•-actin (C4, Santa Cruz, 1:5000 dilution) was used as control. ECL signal was recorded on the ChemiDoc XRS Biorad Imager and data were analysed with Image Lab (Biorad).

### Competition Assay.

HEK293T cells expressing Flag-STING were seeded in 12-well plates and incubated in serum-free media with C-176-22 in combination with C-178 or C-176-09 at indicated concentrations for 30 min. Cells were harvested in PBS and analysed by in-gel analysis of C-176-22 labelling STING (see below).

### Gel-based analysis of BSF0051 binding STING.

HEK293T cells expressing Flag-STING were incubated with BSF0051 (0.5 µM) in serum free media, collected in PBS and lysed by repetitive freezing and thawing. 43 µl of lysed cells were treated with freshly prepared "click reagent" mixture containing tris(benzyltriazolylmethyl)amine (TBTA) (3 µl/sample, 3 mM in 1:4 DMSO:t-ButOH), tetramethylrhodamine (TAMRA) azide (2 µl/sample, 1.25 mM in DMSO), and freshly prepared CuSO4 (1 µl/sample) and tris-(2-carboxyethyl)phosphine hydrochloride (TCEP) (1 µl/sample) and incubated at room temperature for 1 h. The reaction was quenched by addition of reducing sample buffer. In-gel fluorescence was visualized using Fusion FX (Vilber Lourmat) and analysed by Fusion capt advance acquisition software.

### Protein mass spectrometry.

Flag-STING or Flag-STING C91S expression was induced in HEK293T overnight by doxycycline. Cells were treated with or without C-178 or C-176 (1 µM) for 30 min and lysed for in lysis buffer (20 mM Hepes, 150 mM NaCl, 10 % glycerin and 1 % DDM). Flag-STING was immunoprecipitated using anti-Flag M2 affinity gel agarose gel (Sigma) for 2 h at 4° C. Precipitated proteins were eluted using Flag peptide according to the manufacturer's instructions. Protein mass spectrometry was performed on a Shimadzu MS2020 connected to a Nexerra UHPLC system equipped with a Waters ACQUITY UPLC BEH C4 1.7 µm 2.1x50 mm column. Buffer A: 0.05 % HCOOH in H2O Buffer B: 0.05 % HCOOH in acetonitrile. Analytical gradient was from 10 % to 90 % B within 6.0 min with 0.75 ml/min flow. MS was collected from 300-2000 Da and the spectra were deconvoluted using the software MagTran.

### RESULTS

### A high-throughput chemical screen identifies a small molecule inhibitor of the STING signalling pathway

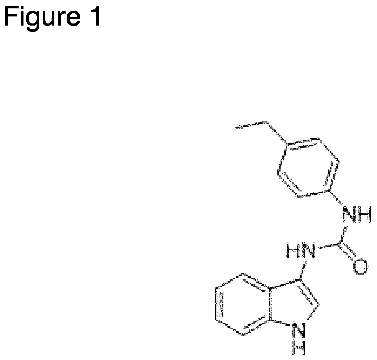

We performed a high-throughput chemical screen in HEK293T cells stably expressing a human STING construct. To stimulate STING, we expressed a construct encoding for cyclic GMP-AMP synthase (cGAS). As a read-out we measured firefly luciferase activity driven by an IFN-• reporter construct. Hits from the primary screen were validated in a counter-screen for their effect on RIG-I driven activation of the IFN-• reporter. Amongst the compounds that selectively suppressed the activation of STING-dependent but not RIG-I-dependent type I IFN induction we identified F2740-0151 (Figure 1).

### Validation of the inhibitory effect of F2740-0151 against STING

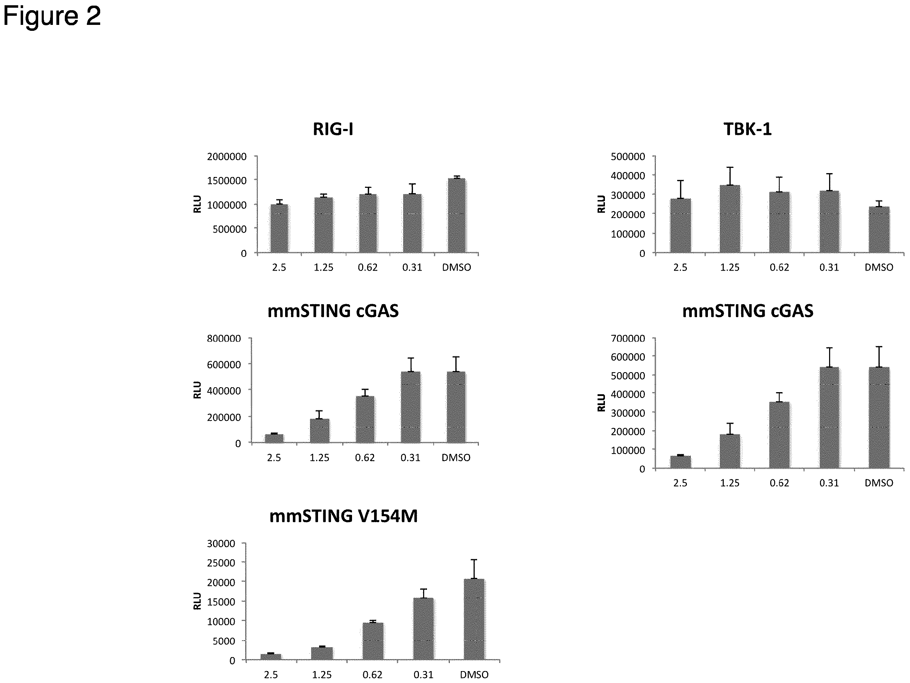

We further validated the effect of F2740-0151 by treating HEK293T cells over expressing TBK1, RIG-I, murine STING with cGAS, human STING with cGAS or murine STING V154M - a hyperactive version of murine STING. Notably, F2740-0151 inhibited the IFN-• reporter activity driven by co-expression of human and murine STING and cGAS or driven by STING V154M in a dose-dependent fashion (Figure 2). However, F2740-0151 failed to suppress RIG-I or TBK1-mediated type I IFN induction. Together this demonstrates, that F2740-0151 is able to specifically interfere with STING-dependent type I IFN responses.

### F2740-0151 blocks STING signalling in the human monocyte cell line THP1

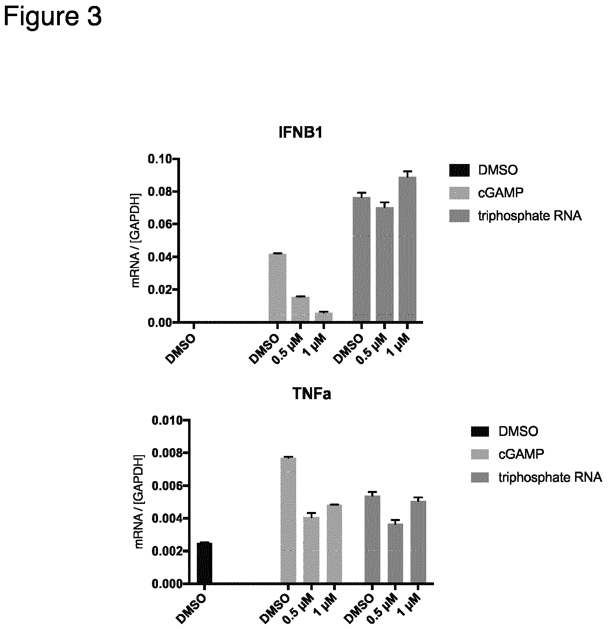

To further validate the inhibitory activity of F2740-0151 against STING, we stimulated human THP1 monocytes with cyclic GMP-AMP (cGAMP), the natural ligand of cGAS, in the presence or absence of F2740-0151. As a control, THP1 cells were stimulated with triphosphate RNA, a ligand for RIG-I. Notably, we observed that F2740-0151 blocked cGAMP-driven type I IFN induction (IFNB1) but not triphosphate-driven type I IFN induction (Figure 3). In addition, we also observed an suppressive effect of F2740-0151 with regards to the cGAMP-triggered expression of TNF-• (Figure 3). Together this indicates that F2740-0151 potently inhibits STING signalling in human cells.

### F2740-0151 directly binds to STING C91

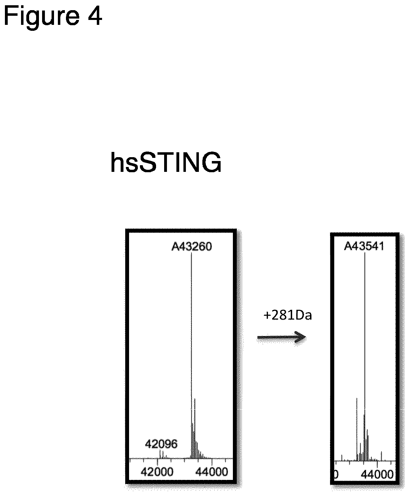

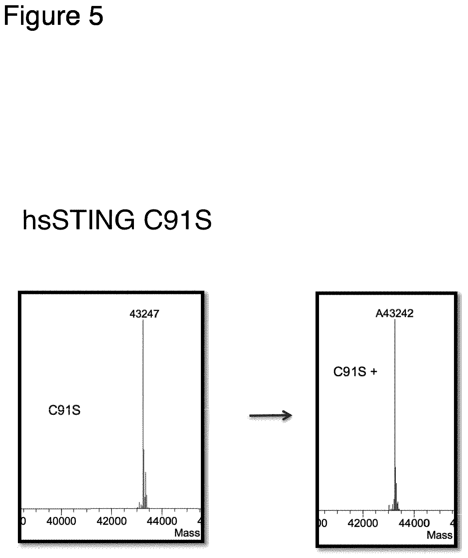

To understand the mechanism of action of F2740-0151, we tested whether the compound may directly bind to STING. To test this, we performed mass spectrometry analysis of STING immunoprecipitated from HEK293T cells stably expressing human or mouse STING in the presence or absence of F2740-0151. Of note, we detected a mass shift of ∼ 280 Da from the samples of those cells treated with F2740-0151 (Figure 4). Previously, we have demonstrated that small molecules can modulate the activity of STING by binding to C91. Thus, we tested whether F2740-0151 may require the presence of Cys91 to bind to STING. Indeed, using a human STING C91S mutant, we observed that F2740-0151 was no longer bound to STING as evidenced by the failure to induce the mass shift observed on the wild-type STING protein (Figure 5). In sum, these data indicate that F2740-0151 modulates the activity of STING by binding to C91.

### A click-chemistry approach to confirm the direct interaction of F2740-0151-L and STING

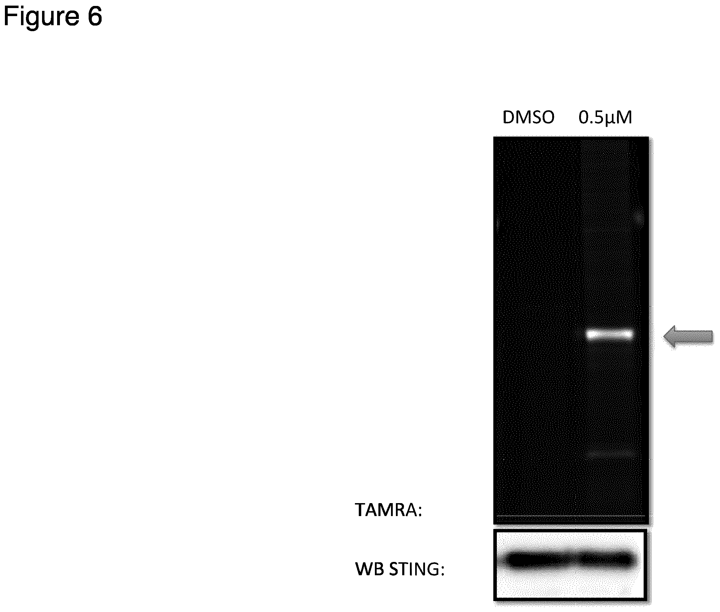

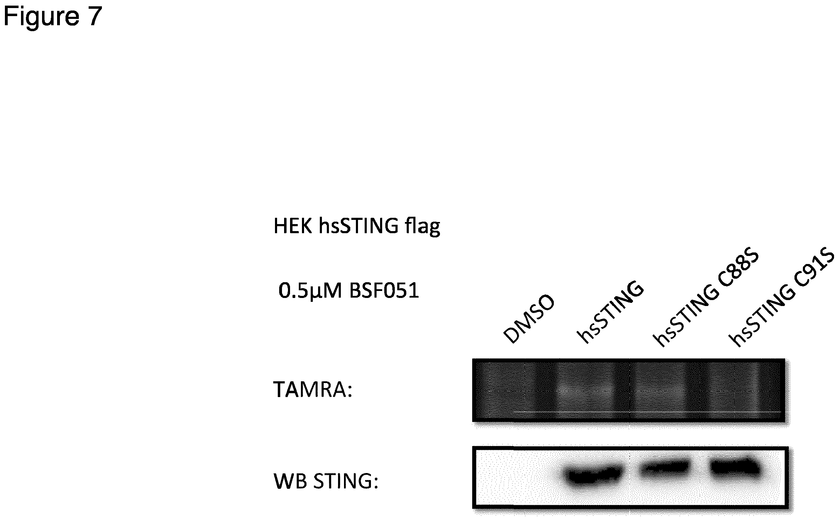

To further validate the direct interaction of F2740-0151 and STING, we synthesized a derivative of F2730-0151, termed F2740-0151-L, which contained an alkyne handle that permits for click-chemistry experiments. HEK293T cells expressing human STING were exposed to F2740-0151-L, lysed, coupled to tetramethylrhodamine (TAMRA-) azide tag and F2740-0151-L-modified proteins were visualized using in-gel fluorescence scanning. Notably, we observed a prominent signal that corresponds to STING that was evident in the samples derived from F2740-0151-L treated cells (Figure 6). The intensity of the signal varied according to the dose of F2740-0151-L that was used for the pre-treatment (Figure 6). These data show that F2740-0151-L binds to STING directly. Finally, we tested the ability of this binding on different human STING mutants. Of note, when tested on the STING C91S mutant, F2740-0151-L did no longer produce a signal (Figure 7). In contrast, no defect in labelling relative to WT STING was observed in cells expressing the STING C88S mutant. Together, this shows that STING is modified on C91 by F2740-0151-L.

### Derivatives of F2740-0151

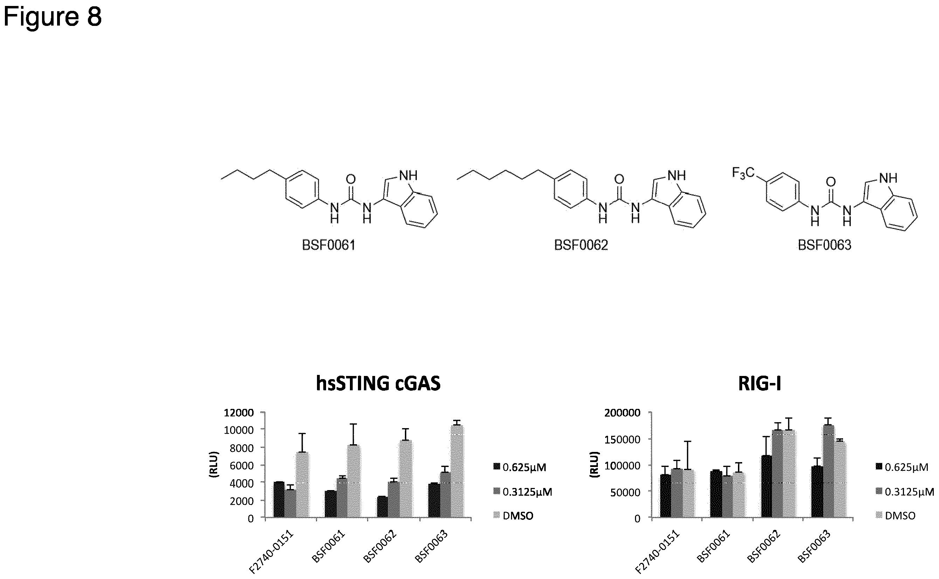

Finally, we synthesized derivatives of F2740-0151 (namely BSF0061, BSF0062 and BSF0063) and assessed their inhibitory potential towards STING. We observed strong inhibition of STING-mediated type I IFN induction of all the analogs presented in Figure 8.

### Chemical synthesis.

All chemical reagents and anhydrous solvents for synthesis were purchased from commercial suppliers (Sigma-Aldrich, Fluka, Acros) and were used without further purification or distillation. The composition of mixed solvents is given by the volume ratio (v/v). 1H and 13C nuclear magnetic resonance (NMR) spectra were recorded on a Bruker DPX 400 (400 MHz for 1H, 100 MHz for 13C, respectively) or Bruker AVANCE III 400 Nanobay (400 MHz for 1H, 100 MHz for 13C, respectively) with chemical shifts (•) reported in ppm relative to the solvent residual signals of DMSO-d6 (2.50 ppm for 1H, 39.52 ppm for 13C). Coupling constants are reported in Hz. LC-MS was performed on a Shimadzu MS2020 connected to a Nexerra UHPLC system equipped with a Waters ACQUITY UPLC BEH C18 1.7µm 2.1x50mm column. Buffer A: 0.05% HCOOH in H2O Buffer B: 0.05% HCOOH in acetonitrile. Analytical gradient was from 10% to 90% B within 6.0 min with 0.5 ml/min flow.

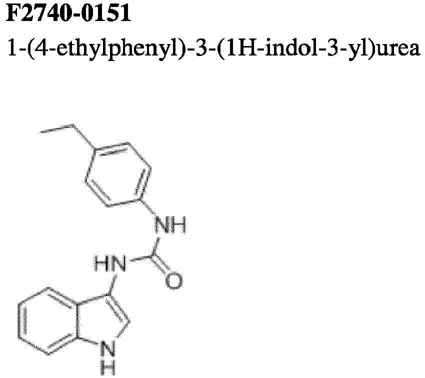

0.187 mL of 4-Ethylaniline (1.0 eq., 1.5 mmoles) and 0.237 g of 3-Isocyanato-1H-indole (1.0 eq., 1.5 mmoles) were dissolved in 1.5 ml DMF and stirred for 2h. The mixture was poured in 80ml of water/AcOEt/Hexane 2:1:1, the phases were separated, the aqueous phase was extracted with 40 ml AcOEt/Hexane 1:1. The combined organic phases were washed with 40 ml water. At this stage a fluffy precipitate formed. it was filtered off and consisted of the product (ca 25 mg). the org phase was dried, and evaporated. the filtered solid was added, the residue was dissolved in MeOH/DCM1:1 and adsorbed on 1.2g Silica. FC: 12g SiO2, Hx to AcOEt yielded the pure compound (330 mg, 79%). ¹H NMR (400 MHz, DMSO) • 10.73 (s, 1 H), 8.49 (s, 1 H), 8.41 (s, 1 H), 7.51 (m, 2 H), 7.41-7.31 (m, 3 H), 7.14-7.07 (m, 3 H), 7.02 (m, 1 H), 2.55 (q, 2 H, *J* = 7.6 Hz), 1.17 (t, 3 H, *J* = 7.6 Hz).

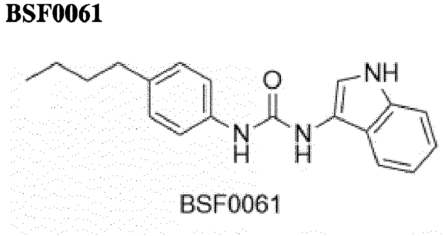

0.048 mL of 4-butylaniline (1.000 eq., 0.32 mmoles) and 0.050 g of 3-Isocyanato-1H-indole (1.0 eq., 0.32 mmoles) were dissolved in 1.0 ml DCM and stirred for 1h. The heterogenous mixture was diluted in MeOH/DCM1:1 (ca 4ml) and adsorbed on 1.5g Silica. FC: 12g SiO2, Hx to AcOEt yielded the pure compound (56 mg, 58%). ¹H NMR (400 MHz, DMSO) • 10.72 (s, 1 H), 8.48 (s, 1 H), 8.41 (s, 1 H), 7.53-7.48 (m, 2 H), 7.40-7.32 (m, 3 H), 7.13-7.07 (m, 3 H), 7.02 (m, 1 H), 2.50 (q, 2 H, *J* = 7.3 Hz), 1.57-1.49 (m, 2 H), 1.30 (m, 2 H), 0.90 (t, 3 H, *J* = 7.3 Hz).

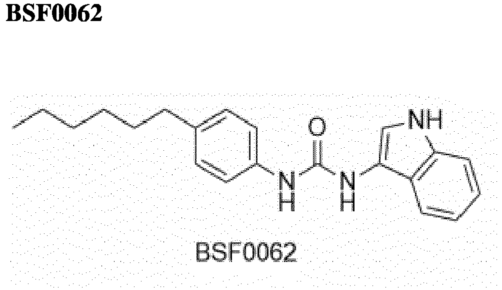

0.056 mL of 4-hexylaniline (1.0 eq., 0.32 mmoles) and 0.050 g of 3-Isocyanato-1H-indole (1.0 eq., 0.32 mmoles) were dissolved in 1.0 ml DCM and stirred for 1h. The heterogenous mixture was diluted in MeOH/DCM1:1 (ca 4ml) and adsorbed on 1.5g Silica. FC: 12g SiO2, Hx to AcOEt yielded the pure compound (74 mg, 71%). ¹H NMR (400 MHz, DMSO) • 10.72 (d, 1 H, *J* = 1.2 Hz), 8.48 (s, 1 H), 8.41 (s, 1 H), 7.53-7.48 (m, 2 H), 7.40-7.32 (m, 3 H), 7.13-7.06 (m, 3 H), 7.02 (m, 1 H), 1.57-1.50 (m, 2 H), 1.28 (m, 6 H), 0.86 (m, 3 H).

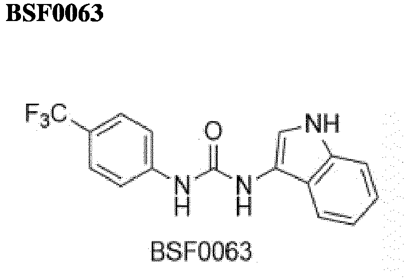

0.039 mL of 4-(trifluoromethyl)aniline (1.0 eq., 0.32 mmoles) and 0.050 g of 3-Isocyanato-1H-indole (1.0 eq., 0.32 mmoles) were dissolved in 1.0 ml DCM and stirred for 1h. The heterogenous mixture was diluted in MeOH/DCM1:1 (ca 4ml) and adsorbed on 1.5g Silica. FC: 12g SiO2, Hx to AcOEt yielded the pure compound (65 mg, 64%). ¹H NMR (400 MHz, DMSO) • 10.80 (d, 1 H), 9.03 (s, 1 H), 8.61 (s, 1 H), 7.69 (m, 2 H), 7.63 (m, 2 H), 7.53 (m, 2 H), 7.35 (d, 1 H, *J* = 8.1 Hz), 7.11 (m, 1 H), 7.03 (m, 1 H).

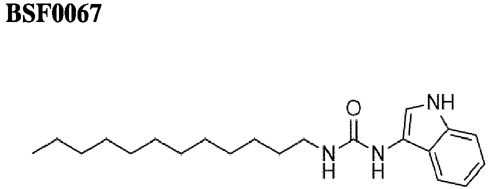

0.059 g of dodecylamine (1.0 eq., 0.32 mmoles) and 0.050 g of 3-Isocyanato-1H-indole (1.0 eq., 0.32 mmoles) were dissolved in 1.0 ml DCM and stirred for 4h. The thick suspension was diluted with DCM (ca 2ml), filtered and washed with DCM (79 mg, 73%). ¹H NMR (400 MHz, DMSO) • 10.60 (s, 1 H), 8.06 (s, 1 H), 7.47 (d, 1 H, *J* = 7.9 Hz), 7.38 (d, 1 H, *J* = 2.3 Hz), 7.30 (d, 1 H, *J* = 8.1 Hz), 7.06 (t, 1 H, *J* = 7.0 Hz), 6.97 (m, 1 H), 5.97 (t, 1 H, *J* = 5.6 Hz), 3.08 (q, 2 H, *J* = 6.6 Hz), 1.44-1.41 (m, 2 H), 1.25 (s, 18 H), 0.86 (m, 3 H, *J* = 6.5 Hz).

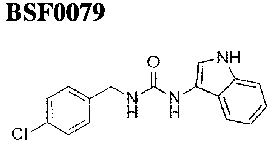

0.039 ml of 4-chlorobenzylamine (1.0 eq., 0.32 mmoles) and 0.050 g of 3-Isocyanato-1H-indole (1.0 eq., 0.32 mmoles) were dissolved in 1.0 ml DCM and stirred for 4h. The suspension was filtered and the solid washed with DCM/hexane 1:1 (2ml) (66 mg, 70%). ¹H NMR (400 MHz, DMSO) • 10.65 (s, 1 H), 8.26 (s, 1 H), 7.49 (d, 1 H, *J* = 7.8 Hz), 7.43-7.27 (m, 6 H), 7.07 (m, 1 H), 6.98 (m, 1 H), 6.53 (t, 1 H, *J* = 6.0 Hz), 4.30 (d, 2 H, *J* = 6.0 Hz).

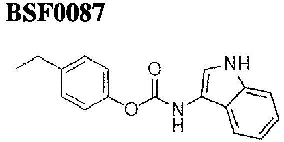

0.039 g of 4-ethylphenol (1.0 eq., 0.32 mmoles) and 0.050 g of 3-Isocyanato-1H-indole (1.0 eq., 0.32 mmoles) were dissolved in 1.0 ml DCM and stirred for 4h. The suspension was filtered and the solid washed with DCM/hexane 1:1 (2ml) (45 mg, 51%). ¹H NMR (400 MHz, DMSO) • 10.84 (s, 1 H), 9.97 (s, 1 H), 7.79 (d, 1 H, *J* = 7.9 Hz), 7.44 (d, 1 H, *J* = 2.3 Hz), 7.34 (d, 1 H, *J* = 8.2 Hz), 7.26 (m, 2 H), 7.17-7.07 (m, 3 H), 6.99 (t, 1 H, *J* = 7.4 Hz), 2.63 (q, 2 H, *J* = 7.5 Hz), 1.20 (t, 3 H, *J* = 7.6 Hz).

### REFERENCES

1 Ishikawa, H., Ma, Z. & Barber, G. N. STING regulates intracellular DNA-mediated, type I interferon-dependent innate immunity. Nature 461, 788-792, doi:10.1038/nature08476 (2009).
2 Ishikawa, H. & Barber, G. N. STING is an endoplasmic reticulum adaptor that facilitates innate immune signalling. Nature 455, 674-678, doi:10.1038/nature07317 (2008).
3 Sun, L., Wu, J., Du, F., Chen, X. & Chen, Z. J. Cyclic GMP-AMP synthase is a cytosolic DNA sensor that activates the type I interferon pathway. Science 339, 786-791, doi:10.1126/science.1232458 (2013).
4 Wu, J. et al. Cyclic GMP-AMP is an endogenous second messenger in innate immune signaling by cytosolic DNA. Science 339, 826-830, doi: 10.1126/science. 1229963 (2013).
5 Gao, P. et al. Cyclic [G(2',5')pA(3',5')p] is the metazoan second messenger produced by DNA-activated cyclic GMP-AMP synthase. Cell 153, 1094-1107, doi:10.1016/j.cell.2013.04.046 (2013).
6 Zhang, X. et al. Cyclic GMP-AMP containing mixed phosphodiester linkages is an endogenous high-affinity ligand for STING. Molecular cell 51, 226-235, doi:10.1016/j.molcel.2013.05.022 (2013).
7 Diner, E. J. et al. The innate immune DNA sensor cGAS produces a noncanonical cyclic dinucleotide that activates human STING. Cell reports 3, 1355-1361, doi:10.1016/j.celrep.2013.05.009 (2013).
8 Burdette, D. L. et al. STING is a direct innate immune sensor of cyclic di-GMP. Nature 478, 515-518, doi:10.1038/nature10429 (2011).
9 Cavlar, T., Deimling, T., Ablasser, A., Hopfner, K. P. & Hornung, V. Species-specific detection of the antiviral small-molecule compound CMA by STING. The EMBO journal 32, 1440-1450, doi:10.1038/emboj.2013.86 (2013).
10 Conlon, J. et al. Mouse, but not human STING, binds and signals in response to the vascular disrupting agent 5,6-dimethylxanthenone-4-acetic acid. Journal of immunology 190, 5216-5225, doi:10.4049/jimmunol.1300097 (2013).
11 Huang, Y. H., Liu, X. Y., Du, X. X., Jiang, Z. F. & Su, X. D. The structural basis for the sensing and binding of cyclic di-GMP by STING. Nature structural & molecular biology 19, 728-730, doi:10.1038/nsmb.2333 (2012).
12 Shang, G. et al. Crystal structures of STING protein reveal basis for recognition of cyclic di-GMP. Nature structural & molecular biology 19, 725-727, doi:10.1038/nsmb.2332 (2012).
13 Shu, C., Yi, G., Watts, T., Kao, C. C. & Li, P. Structure of STING bound to cyclic di-GMP reveals the mechanism of cyclic dinucleotide recognition by the immune system. Nature structural & molecular biology 19, 722-724, doi:10.1038/nsmb.2331 (2012).
14 Yin, Q. et al. Cyclic di-GMP sensing via the innate immune signaling protein STING. Molecular cell 46, 735-745, doi:10.1016/j.molcel.2012.05.029 (2012).
15 Dobbs, N. et al. STING Activation by Translocation from the ER Is Associated with Infection and Autoinflammatory Disease. Cell host & microbe 18, 157-168, doi:10.1016/j.chom.2015.07.001 (2015).
16 Liu, S. et al. Phosphorylation of innate immune adaptor proteins MAVS, STING, and TRIF induces IRF3 activation. Science 347, aaa2630, doi:10.1126/science.aaa2630 (2015).
17 McWhirter, S. M. et al. A host type I interferon response is induced by cytosolic sensing of the bacterial second messenger cyclic-di-GMP. The Journal of experimental medicine 206, 1899-1911, doi:10.1084/jem.20082874 (2009).
18 Abe, T. & Barber, G. N. Cytosolic-DNA-mediated, STING-dependent proinflammatory gene induction necessitates canonical NF-kappaB activation through TBK1. Journal of virology 88, 5328-5341, doi:10.1128/JVI.00037-14 (2014).
19 Konno, H., Konno, K. & Barber, G. N. Cyclic dinucleotides trigger ULK1 (ATG1) phosphorylation of STING to prevent sustained innate immune signaling. Cell 155, 688-698, doi:10.1016/j.cell.2013.09.049 (2013).
20 Crow, Y. J. & Manel, N. Aicardi-Goutieres syndrome and the type I interferonopathies. Nature reviews. Immunology 15, 429-440, doi:10.1038/nri3850 (2015).
21 Liu, Y. et al. Activated STING in a vascular and pulmonary syndrome. The New England journal of medicine 371, 507-518, doi:10.1056/NEJMoa1312625 (2014).
22 Jeremiah, N. et al. Inherited STING-activating mutation underlies a familial inflammatory syndrome with lupus-like manifestations. The Journal of clinical investigation 124, 5516-5520, doi:10.1172/JCI79100 (2014).
23 Gall, A. et al. Autoimmunity initiates in nonhematopoietic cells and progresses via lymphocytes in an interferon-dependent autoimmune disease. Immunity 36, 120-131, doi:10.1016/j.immuni.2011.11.018 (2012).
24 Konig, N. et al. Familial chilblain lupus due to a gain-of-function mutation in STING. Annals of the rheumatic diseases 76, 468-472, doi:10.1136/annrheumdis-2016-209841 (2017).
25 Yan, N. Immune Diseases Associated with TREX1 and STING Dysfunction. Journal of interferon & cytokine research : the official journal of the International Society for Interferon and Cytokine Research 37, 198-206, doi:10.1089/jir.2016.0086 (2017).
26 Ahn, J., Gutman, D., Saijo, S. & Barber, G. N. STING manifests self DNA-dependent inflammatory disease. Proceedings of the National Academy of Sciences of the United States of America 109, 19386-19391, doi:10.1073/pnas.1215006109 (2012).
27 Ahn, J. et al. Inflammation-driven carcinogenesis is mediated through STING. Nature communications 5, 5166, doi:10.1038/ncomms6166 (2014).
28 Ablasser, A. et al. Cell intrinsic immunity spreads to bystander cells via the intercellular transfer of cGAMP. Nature 503, 530-534, doi:10.1038/naturel2640 (2013).
29 Tanaka, Y. & Chen, Z. J. STING specifies IRF3 phosphorylation by TBK1 in the cytosolic DNA signaling pathway. Science signaling 5, ra20, doi:10.1126/scisignal.2002521 (2012).
30 Gao, P. et al. Structure-function analysis of STING activation by c[G(2',5')pA(3',5')p] and targeting by antiviral DMXAA. Cell 154, 748-762, doi:10.1016/j.cell.2013.07.023 (2013).
31 Mukai, K. et al. Activation of STING requires palmitoylation at the Golgi. Nature communications 7, 11932, doi:10.1038/ncomms11932 (2016).
32 Blaskovic, S., Blanc, M. & van der Goot, F. G. What does S-palmitoylation do to membrane proteins? The FEBS journal 280, 2766-2774, doi:10.1111/febs.12263 (2013).
33 Stetson, D. B., Ko, J. S., Heidmann, T. & Medzhitov, R. Trex1 prevents cell-intrinsic initiation of autoimmunity. Cell 134, 587-598, doi:10.1016/j.cell.2008.06.032 (2008).
34 Gray, E. E., Treuting, P. M., Woodward, J. J. & Stetson, D. B. Cutting Edge: cGAS Is Required for Lethal Autoimmune Disease in the Trexl-Deficient Mouse Model of Aicardi-Goutieres Syndrome. Journal of immunology 195, 1939-1943, doi:10.4049/jimmunol.1500969 (2015).
35 Gao, D. et al. Activation of cyclic GMP-AMP synthase by self-DNA causes autoimmune diseases. Proceedings of the National Academy of Sciences of the United States of America 112, E5699-5705, doi:10.1073/pnas.1516465112 (2015).
36 Crowl, J. T., Gray, E. E., Pestal, K., Volkman, H. E. & Stetson, D. B. Intracellular Nucleic Acid Detection in Autoimmunity. Annual review of immunology 35, 313-336, doi:10.1146/annurev-immunol-051116-052331 (2017).

## Claims

**1.** A compound of formula (I): 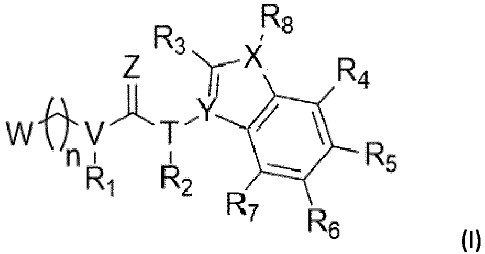 racemates, enantiomers, diastereomers and pharmaceutically acceptable salts thereof, wherein
R¹ and R² are each independently selected from H, C₁-C₁₀ alkyl, C₂-C₆ alkenyl, C₁-C₁₀ haloalkyl or a lone pair of electrons;
R³ is H;
R⁴, R⁵ , R⁶ and R⁷ are each independently selected from H, C₁-C₁₀ alkyl, C₂-C₁₀ alkenyl, C₂-C₁₀ alkynyl, C₁-C₁₀ haloalkyl, C₁-C₁₀ alkoxy, C₁-C₁₀ carboxyalkyl, carboxyl, halogen, nitro, cyano, amino, amido, or ether groups;
R⁸ is selected from H, C₁-C₆ alkyl, C₂-C₆ alkenyl, C₂-C₁₀ alkynyl or C₁-C₆ haloalkyl;
X is selected from O, S, or NH;
Z is selected from O, S, or N;
Y is C;
T and V are independently selected from O, S, or N;
W is selected from saturated or unsaturated C₁-C₂₀ alkyl group, C₆-C₃₁ aryl group, C₆-C₃₁ heteroaryl group, C₅-C₃₁ aryloxy group, or C₅-C₃₁ aralkyl group, wherein each group is optionally substituted with 1, 2, 3 substituents independently selected from -O-CF₃, -CF₃, -O-CHF₂, halogen, -OH, -NH₂, -CN, -OCH₃, C₁-C₆ alkyl, allyl, -C(O)OR¹, -C(O)NH₂, propargyl, -NO₂, -S(O)₂NH₂, and -S(O)CH₃;
and
n is an integer between 0-2,
with the proviso that 1-(4-ethylphenyl)-3-(1H-indol-3-yl)urea is excluded.

**2.** The compound of formula I according to claim 1, wherein it is selected from the group comprising 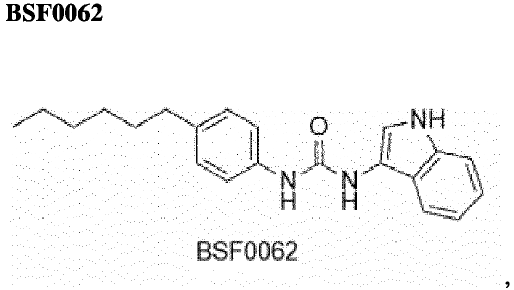 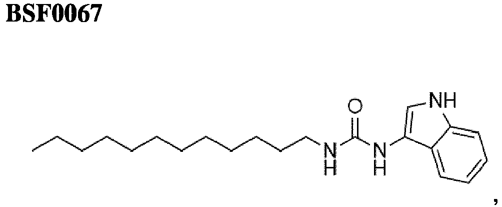 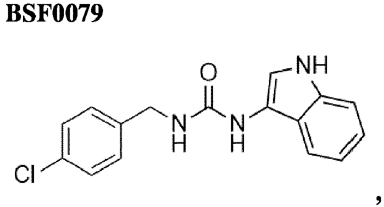 and

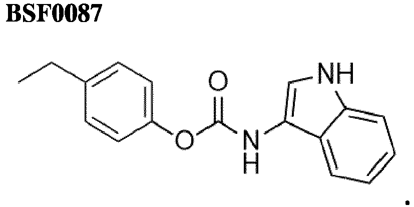

**3.** A compound having formula (I): 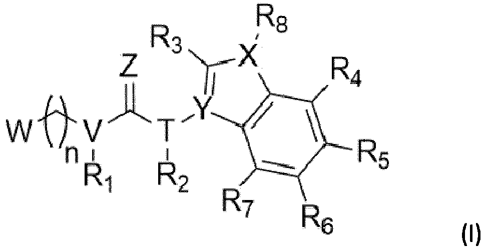 racemates, enantiomers, diastereomers and pharmaceutically acceptable salts thereof, wherein
R¹ and R² are each independently selected from H, C₁-C₁₀ alkyl, C₂-C₆ alkenyl, C₁-C₁₀ haloalkyl or a lone pair of electrons;
R³ is H;
R⁴, R⁵ , R⁶ and R⁷ are each independently selected from H, C₁-C₁₀ alkyl, C₂-C₁₀ alkenyl, C₂-C₁₀ alkynyl, C₁-C₁₀ haloalkyl, C₁-C₁₀ alkoxy, C₁-C₁₀ carboxyalkyl, carboxyl, halogen, nitro, cyano, amino, amido, or ether groups;
R⁸ is selected from H, C₁-C₆ alkyl, C₂-C₆ alkenyl, C₂-C₁₀ alkynyl or C₁-C₆ haloalkyl;
X is selected from O, S, or NH;
Z is selected from O, S, or N;
Y is C;
T and V are independently selected from O, S, or N;
W is selected from saturated or unsaturated C₁-C₂₀ alkyl group, C₆-C₃₁ aryl group, C₆-C₃₁ heteroaryl group, C₅-C₃₁ aryloxy group, or C₅-C₃₁ aralkyl group, wherein each group is optionally substituted with 1, 2, 3 substituents independently selected from -O-CF₃, -CF₃, -O-CHF₂, halogen, -OH, -NH₂, -CN, -OCH₃, C₁-C₆ alkyl, allyl, -C(O)OR¹, -C(O)NH₂, propargyl, -NO₂, -S(O)₂NH₂, and -S(O)CH₃; and n is an integer between 0-2,
for use in the treatment of a STING associated disease.

**4.** The compound for use according to claim 3, wherein the STING associated disease is Type I interferonopathy.

**5.** The compound for use according to claim 3 or 4, wherein the STING associated disease is selected from the group comprising a cancer, a neurological disorder, an autoimmune disease, hepatitis B, uveitis, cardiovascular disease, age-related macular degeneration and mucositis.

**6.** The compound for use according to anyone of claims 3 to 5, wherein it is selected from the group comprising 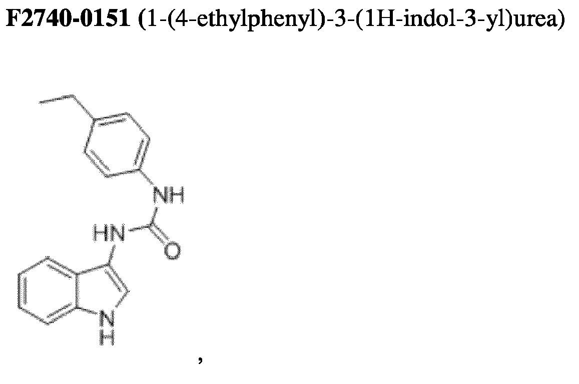 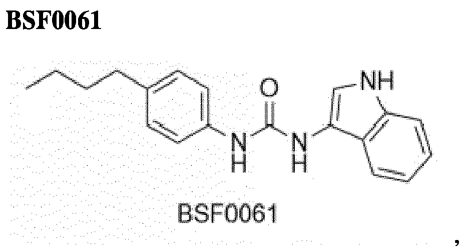 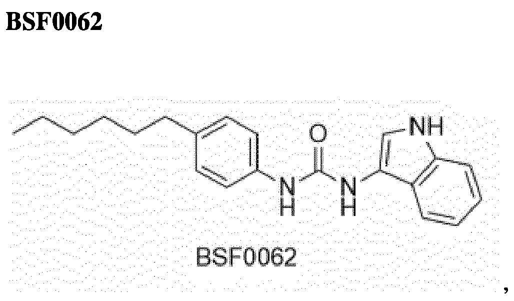 and 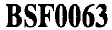 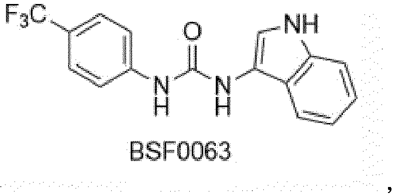 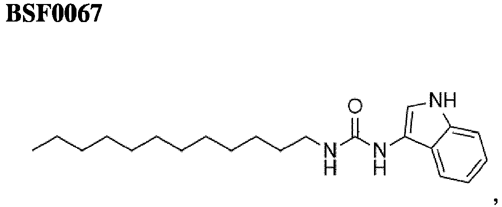 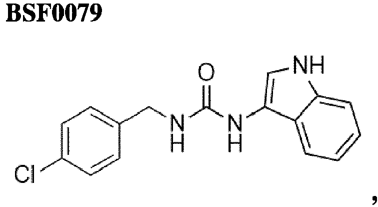 **and**

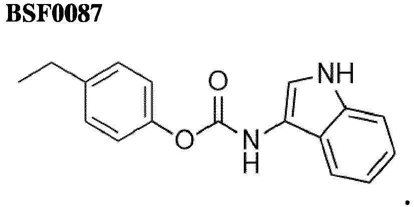

**7.** A pharmaceutical composition comprising a compound of formula (I): 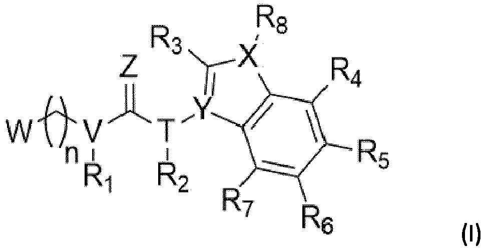 racemates, enantiomers, diastereomers and pharmaceutically acceptable salts thereof, wherein
R¹ and R² are each independently selected from H, C₁-C₁₀ alkyl, C₂-C₆ alkenyl, C₁-C₁₀ haloalkyl or a lone pair of electrons;
R³ is H;
R⁴, R⁵, R⁶ and R⁷ are each independently selected from H, C₁-C₁₀ alkyl, C₂-C₁₀ alkenyl, C₂-C₁₀ alkynyl, C₁-C₁₀ haloalkyl, C₁-C₁₀ alkoxy, C₁-C₁₀ carboxyalkyl, carboxyl, halogen, nitro, cyano, amino, amido, or ether groups;
R⁸ is selected from H, C₁-C₆ alkyl, C₂-C₆ alkenyl, C₂-C₁₀ alkynyl or C₁-C₆ haloalkyl;
X is selected from O, S, or NH;
Z is selected from O, S, or N;
Y is C;
T and V are independently selected from O, S, or N;
W is selected from saturated or unsaturated C₁-C₂₀ alkyl group, C₆-C₃₁ aryl group, C₆-C₃₁ heteroaryl group, C₅-C₃₁ aryloxy group, or C₅-C₃₁ aralkyl group, wherein each group is optionally substituted with 1, 2, 3 substituents independently selected from -O-CF₃, -CF₃, -O-CHF₂, halogen, -OH, -NH₂, -CN, -OCH₃, C₁-C₆ alkyl, allyl, -C(O)OR¹, -C(O)NH₂, propargyl, -NO₂, -S(O)₂NH₂, and -S(O)CH₃; and n is an integer between 0-2, and a pharmaceutically acceptable excipient.

**8.** The pharmaceutical composition of claim 7, wherein the compound is selected from the group comprising 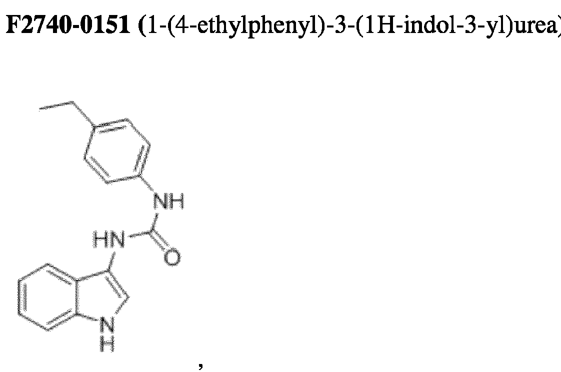 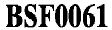 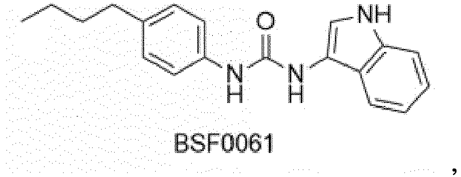 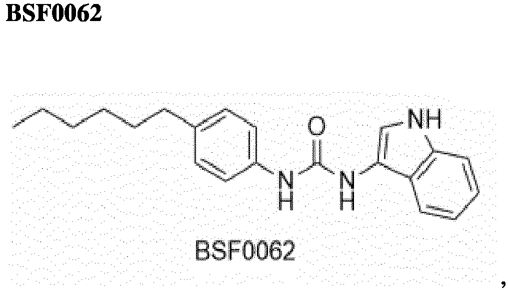 and 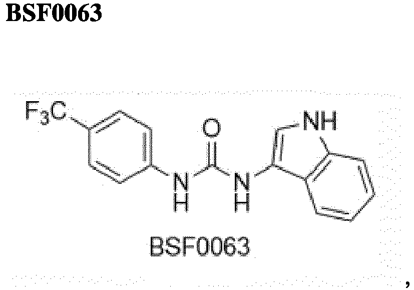 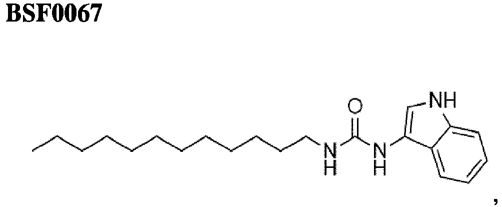 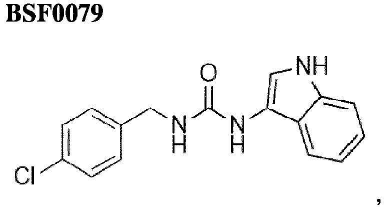 and

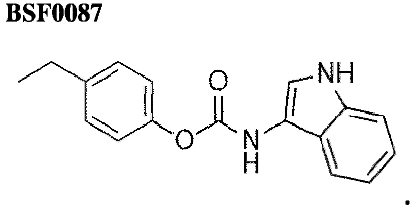

**9.** Use of a compound having formula (I): 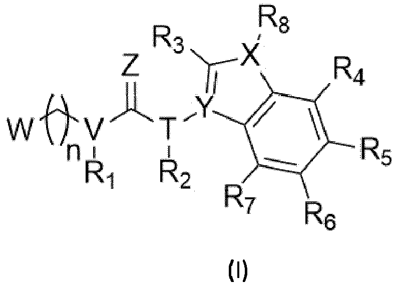 racemates, enantiomers, diastereomers and pharmaceutically acceptable salts thereof, wherein
R¹ and R² are each independently selected from H, C₁-C₁₀ alkyl, C₂-C₆ alkenyl, C₁-C₁₀ haloalkyl or a lone pair of electrons;
R³ is H;
R⁴, R⁵, R⁶ and R⁷ are each independently selected from H, C₁-C₁₀ alkyl, C₂-C₁₀ alkenyl, C₂-C₁₀ alkynyl, C₁-C₁₀ haloalkyl, C₁-C₁₀ alkoxy, C₁-C₁₀ carboxyalkyl, carboxyl, halogen, nitro, cyano, amino, amido, or ether groups;
R⁸ is selected from H, C₁-C₆ alkyl, C₂-C₆ alkenyl, C₂-C₁₀ alkynyl or C₁-C₆ haloalkyl;
X is selected from O, S, or NH;
Z is selected from O, S, or N;
Y is C;
T and V are independently selected from O, S, or N;
W is selected from saturated or unsaturated C₁-C₂₀ alkyl group, C₆-C₃₁ aryl group, C₆-C₃₁ heteroaryl group, C₅-C₃₁ aryloxy group, or C₅-C₃₁ aralkyl group, wherein each group is optionally substituted with 1, 2, 3 substituents independently selected from -O-CF₃, -CF₃, -O-CHF₂, halogen, -OH, -NH₂, -CN, -OCH₃, C₁-C₆ alkyl, allyl, -C(O)OR¹, -C(O)NH₂, propargyl, -NO₂, -S(O)₂NH₂, and -S(O)CH₃; and
n is an integer between 0-2, or pharmaceutical composition of claim 7 or 8, in the manufacture of a medicament.

**10.** The use according to claim 9, wherein the medicament is for the treatment of a STING associated disease.

**11.** The use according to claim 9 or 10, wherein the compound is selected from the group comprising 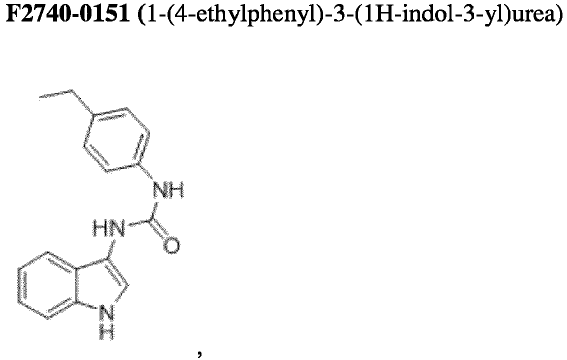 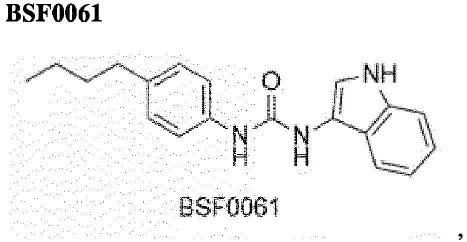 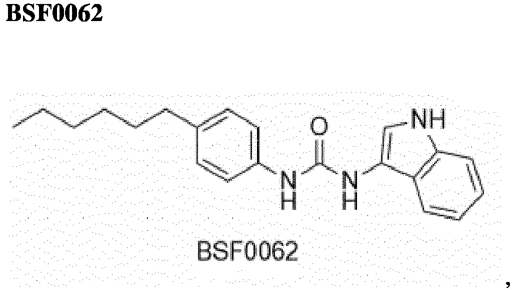 and 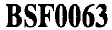 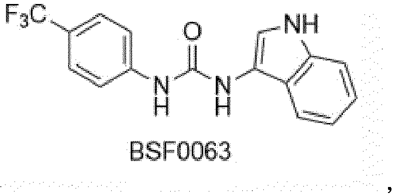 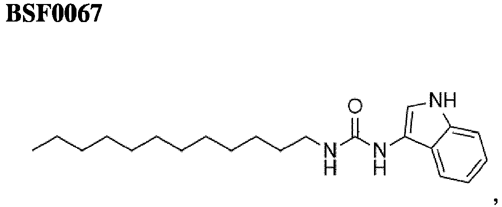 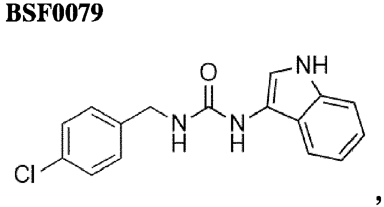 and

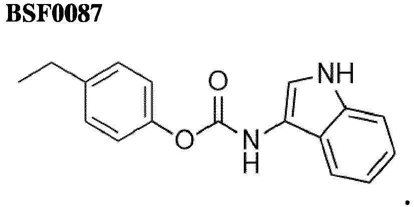

**11.** A kit comprising (1) a pharmaceutical composition of claim 7 or 8, and (2) instructions for use.

**12.** A method of treatment and/or prevention of a STING associated disease comprising administering a pharmaceutical composition of claim 7 or 8.
